# EUROPÄISCHE PATENTSCHRIFT

(11) **EP 2 991 608 B1**
(45) Veröffentlichungstag und Bekanntmachung des Hinweises auf die Patenterteilung: **13.03.2019**
(21) Anmeldenummer: 14723995.8
(22) Anmeldetag: 25.04.2014
(51) Int. Cl.: A61H 1/02

(54) **EXTENSIONSLIEGENVORRICHTUNG ZUR DEKOMPRESSION DER WIRBELSÄULE UND/ODER ZUR DEKOMPRESSION VON GELENKEN**
EXTENSION BED DEVICE FOR DECOMPRESSION OF THE SPINAL COLUMN AND/OR FOR DECOMPRESSION OF JOINTS
DISPOSITIF D'EXTENSION EN DÉCUBITUS POUR LA DÉCOMPRESSION DE LA COLONNE VERTÉBRALE ET/OU POUR LA DÉCOMPRESSION D'ARTICULATIONS

(30) Priorität: 29.04.2013 DE 102013007314
(43) Veröffentlichungstag der Anmeldung: 09.03.2016
(73) Patentinhaber: Heiko, Diedrich, 50259 Pulheim (DE)
(72) Erfinder: Heiko, Diedrich, 50259 Pulheim (DE)
(74) Vertreter: Kirschner, Sebastian
(86) Internationale Anmeldenummer: PCT/EP2014/001102
(87) Internationale Veröffentlichungsnummer: WO 2014/177259

(56) Entgegenhaltungen:
- EP-A1- 1 792 597
- WO-A1-2013/054992
- DE-A1- 2 552 777
- US-A- 1 684 459
- US-A- 4 258 445
- US-A1- 2003 055 456
- US-B1- 6 681 770

## Beschreibung

Die Erfindung betrifft eine Extensionsliegenvorrichtung zur Dekompression der Wirbelsäule und/oder zur Dekompression von Gelenken eines Patienten, mit einem Liegenbereich und mit einem Antriebssystem, wobei der Liegenbereich mehrere Segmente aufweist, wobei der Patient auf dem Liegenbereich anordbar ist, wobei zumindest eines der Segmente mit dem Antriebssystem derart bewegbar ist, so dass zur Dekompression eine Zugkraft und eine Gegenzugkraft auf den Patienten ausübbar ist.

Solche Extensionsliegenvorrichtungen werden zur Behandlung von Rücken- und/oder Gelenkbeschwerden eingesetzt. Der Einsatz solcher Extensionsliegenvorrichtungen ist jedoch nicht auf die Behandlung von Patienten mit Beschwerden beschränkt. Ferner können Patienten bzw. Menschen ohne Beschwerden solche Extensionsliegen im Rahmen einer ganzheitlichen Wirbelsäulen- und/oder Gelenkbehandlung nutzen, um mittels der Dekompression einen regenerierenden, entspannenden und/oder gesundheitsfördernden Effekt zu erzielen. Die Extensionsliegenvorrichtung kann damit zur Prävention, Akuttherapie und Rehabilitation von bandscheiben- und/oder gelenkbedingten Erkrankungen und/oder Verletzungen eingesetzt werden.

Aus der gattungsgemäßen WO 2004/058124 A2 ist eine Extensionsliegenvorrichtung zur spinalen Dekompression der Wirbelsäule mit einem Liegenbereich und mit einem Antriebssystem bekannt. Der Liegenbereich weist im Wesentlichen drei Segmente auf, nämlich ein kippbares Kopfsegment zur Ablage des Kopfes des Patienten, ein feststehendes, oberes Liegensegment und ein verschiebbares, unteres Liegensegment. Das untere Liegensegment umfasst einen Hüftablagebereich, der eine Kippung des Beckens ermöglicht. Zwischen dem oberen Liegensegment und dem Hüftablagebereich sind zwei aufragende Armteile vorgesehen, um den Rumpf des Patienten seitlich zu greifen. Die zwei Armteile, die den Körper des Patienten seitlich umfassen, können mit einer Handkurbel in eine entsprechende Greifstellung verfahren werden. Die beiden Armteile klemmen den Körper des Patienten im Wesentlichen in Höhe des Beckenkamms beidseitig des Beckens des Patienten ein. Um besonders große oder korpulente Patienten zu behandeln, ist ein weiteres Halterungssystem vorgesehen, wobei statt der Armteile ein Hüftgurt am Beckenbereich des Patienten angelegt wird und dieser Hüftgurt mit dem unteren Liegensegment verbunden wird. Hierdurch ist das Becken des Patienten mit dem verschiebbaren, unteren Liegensegment verbunden. Mit dem oberen Liegensegment wird der Patient über ein Gurtsystem verbunden, dass den Brustkorb und/oder den Bauchraum des Patienten umfasst. Das Gurtsystem weist ferner entsprechende Armschlaufen auf, die von den Armen des Patienten durchgriffen werden. Mittels eines Antriebssystems ist das untere Liegensegment relativ zum feststehenden, oberen Liegensegment verschiebbar. Das Antriebssystem umfasst eine Gleitschiene, an der das untere Liegensegment verschiebbar gelagert ist. Durch die Betätigung eines Elektromotors kann das untere Liegensegment in der horizontalen Ebene verfahren werden. Mit dem Kopfsegment soll die Halswirbelsäule gezielt behandelbar sein. Das Kopfsegment und damit der Kopf des Patienten kann dabei derart gekippt werden, so dass der entsprechend zu therapierende Halswirbelbereich auseinander gezogen werden kann. Der Hüftablagebereich kann in mehreren Stufen relativ zur Horizontalen eingestellt werden, nämlich im Winkel 0°, 5°, 10°, 15°, 20° und 25° Grad, um das Becken des Patienten zu kippen. Um eine Zugkraft auf Wirbelpaare L5-S1 auszuüben, wird der Hüftablagebereich im Winkel von 0° Grad eingestellt. Die Einstellung im Winkel von 5° Grad wird genutzt, um das Wirbelpaar L5-S1 und L4-L5 zu therapieren. Die Einstellung von 10° Grad wird genutzt, um Wirbelpaar L4-L5 zu therapieren. Den anderen Wirbelpaaren sind die weiteren Winkel zugeordnet. Ein Infrarot-Heizelement ist in die Extensionsliegenvorrichtung integriert, um die Muskulatur des Patienten zu erwärmen und dadurch die Muskeln zu entspannen.

Eine weitere Extensionsliegenvorrichtung mit der Möglichkeit, einen Patienten mit entsprechenden Gurtsystemen mit einem Liegenbereich zu fixieren, ist bspw. aus der US 2011/027031 A1 oder WO2013054992A1 bekannt. Die gattungsgemäße Extensionsliegenvorrichtung ist noch nicht optimal ausgebildet. Durch die Winkelverstellung des Hüftablagebereiches soll die Lendenwirbelsäule in eine geeignete Strecklage gebracht werden. Zwar lässt sich die bei der Streckung angewendete Kraft dosieren, aber der Winkel, unter dem die Zugkraft und die entsprechende Gegenzugkraft auf die Wirbel wirken, ist hierbei nicht exakt bestimmbar. Mit der gattungsgemäßen Extensionsliegenvorrichtung erfolgt eine Zugkrafteinleitung unter den Achseln und an der Hüfte, wodurch eine Streckung des gesamten Lenden- und Brustwirbelbereiches erfolgt. Es werden mit der gattungsgemäßen Extensionsliegenvorrichtung Wirbelsäulenbereiche mit jeweils mehreren Bandscheiben und Wirbelgelenkpaaren gestreckt. Da der Eingriffswinkel der Kraft nicht eindeutig einstellbar ist und die Wirbelsäule S-förmig geschwungen ist, greifen an den einzelnen Wirbeln des gestreckten Bereiches unterschiedliche Kraftkomponenten der Zugkräfte an. Hierdurch ist es nicht möglich, an allen Bandscheiben des entsprechenden Wirbelsäulenbereiches gleiche Entlastungen zu erzeugen oder gezielt auf eine einzelne Bandscheibe zu wirken. Eine individuelle Einstellung des Liegenbereiches an die unterschiedlichen Körpergrößen des Patienten ist dabei nur sehr eingeschränkt möglich.

Ferner weist die gattungsgemäße Extensionsliegenvorrichtung die weiteren folgenden Nachteile auf. Von einem großen Anteil der Patienten werden das Gurtsystem und/oder die Armteile als unangenehm empfunden, da durch das Gurtsystem und/oder die Armteile lokale, schmerzhafte Druckstellen auftreten können. Das Becken und der Bauch werden zusammengepresst. Durch das den Brustkorb und/oder den Bauchraum umfassende Gurtsystem wird die Brust-/Bauchatmung des Patienten behindert. Teilweise muss die Therapie sogar abgebrochen werden.

Ein weiterer Nachteil der gattungsgemäßen Extensionsliegenvorrichtung ist, dass der Patient auf die Liege aufsteigen muss, um therapiert zu werden. Da der Patient zumeist akute Rückenschmerzen hat, fällt dem Patienten das Aufsteigen, das Hinlegen und insbesondere das Aufstehen schwer.

Aus der US 6,428,497 B1 ist eine Extensionsliegenvorrichtung zur Dekompression der Wirbelsäule bekannt. Die Extensionsliegenvorrichtung weist einen Liegenbereich und ein Antriebssystem auf. Der Liegenbereich weist mehrere Segmente auf. Es sind ein Kopfsegment, ein Fußsegment und ein Hüftsegment vorgesehen. Der Patient ist auf diesen Segmenten anordbar. Das Kopfsegment besteht aus einem Grundkörper und zwei Kopfhalterungen, die verschieblich an dem Grundkörper angeordnet sind, so dass der Kopf seitlich von den Kopfhalterungen gehalten wird. Das Hüftsegment ist als verschiebbarer Schlitten ausgestaltet, wobei der Schlitten zwei seitliche Halterungen zur Halterung der Hüfte des Patienten aufweist. Der Patient wird nun bei der Behandlung zwischen den beiden Halterungen am Kopf und an der Hüfte eingeklemmt. Der Schlitten wird danach mittels des Antriebssystems verschoben, um die Wirbelsäule zu strecken und zur Dekompression eine Zugkraft und eine Gegenzugkraft auf den Patienten auszuüben. Diese Extensionsliegenvorrichtung kommt zwar ohne Gurte aus, aber das Einklemmen des Patienten wird dennoch von dem Patienten als unangenehm empfunden.

Aus der US 6,681,770 B1 ist eine Liege zur Behandlung des Rückens eines Patienten bekannt. Diese Liege weist einen Liegenbereich mit mehreren Segmenten auf. Die Segmente sind unterschiedlich hoch ausgebildet, so dass der Liegenbereich im wesentlichen der Kontur der Wirbelsäule in Längsrichtung folgt. Der Liegenbereich weist einen in Längsrichtung, mittig, im Bereich der Wirbelsäule verlaufenden Schlitz auf. Der Schlitz teilt die Segmente in eine rechte Gruppe und in eine linke Gruppe. Der Liegenbereich ist in der Länge verstellbar. Die Segmente sind mittels eines Scherengitters gleichförmig beabstandbar. Das Beabstanden der Segmente dient lediglich dazu, die Länge des Liegenbereiches auf die Größe des Patienten einzustellen. Die Lage der Segmente ist an die Größe des Patienten anpassbar. Das Scherengitter ist dabei mittels eines Spindeltriebs spreizbar. Ein Behandlungsgerät ist im Schlitz angeordnet und im Schlitz entlang der Wirbelsäule verfahrbar. Das Behandlungsgerät ist zum Beispiel als Rolle zum Kneten der Muskulatur, als Vibrationsgerät oder als Wasserstrahlbehandlungsgerät zur Massage der Muskulatur ausgebildet.

Aus der DE 102 46 760 A1 ist ein zweiteiliges Bett für die Behandlung von Wirbelsäulenleiden während des nächtlichen Schlafes bekannt. Das Bett weist ein festes Segment für den Kopf und den Oberkörper eines Menschen sowie ein bewegliches Segment für Gesäß und die Beine auf. Das bewegliche Segment trägt eine Matratze und ist längsbeweglich auf Rollen abgestützt. Es ist ein Antriebssystem zum Bewegen des beweglichen Segments und zur Erzeugung von sinusartigen Bewegungsabläufen in Längsrichtung vorgesehen. Diese Bewegung des beweglichen Segments soll nachts unterhalb einer Reizschwelle des Patienten während der Schlafphase erfolgen, um den Patienten sanft zu bewegen, ohne ihn dabei aufzuwecken. Das Bett ist nicht zur spinalen Dekompression geeignet, da keine hinreichenden Zugkräfte eingeleitet werden.

Aus der US 6,742,202 B2 ist ein Tragsystem zum Aufbau einer Matratze bekannt. Die Matratze weist eine Vielzahl von aufragenden Stützelementen, nämlich stiftförmige Hülsen auf, wobei die Hülsen mittels jeweils einer Feder federnd gelagert sind. Es sind eine Vielzahl von Hülsen in einem regelmäßigen Raster nebeneinander aufragend angeordnet. Wenn sich nun ein Benutzer auf dieses Matratzensystem legt, so werden die Stützelemente in unterschiedlicher Höhe angeordnet. Die Stützelemente passen sich der Kontur des Körpers an. Mittels der Stützelemente kann der auf der Matratze liegende Körper massiert werden. Die Höhe der Hülsen ist mittels eines Aktors und eines PCs einstellbar. Der Aktor kann hydraulisch, pneumatisch oder elektrisch betätigt sein. Durch diese Hülsenanordnung wird ein Hängematteneffekt verhindert. Eine Dekompression der Wirbelsäule ist nicht möglich.

Aus der US2013/0 019 408 A1 ist ein Patientenbett mit einer Liegefläche bekannt, die eine Vielzahl von aufblasbaren Segmenten aufweist, wobei die Segmente den Patienten abstützen. Die Segmente sind jeweils mit einem Drucksensor gekoppelt. Um ein Wundliegen des Patienten zu verhindern, wird der Druck in den Segmenten in Abhängigkeit von dem gemessenen Druck variiert. Eine Dekompression der Wirbelsäule ist nicht möglich.

Die Erfindung stellt eine Extensionsliegenvorrichtung nach Anspruch 1 bereit. Der Erfindung liegt daher die Aufgabe zugrunde, die gattungsgemäße Extensionsliegenvorrichtung derart auszugestalten und weiterzubilden, so dass die Wirksamkeit der Therapie verbessert ist, das Angebotsspektrum erweitert wird und die Anwendung des Verfahrens für den Patienten angenehmer und effektiver ist.

Diese der Erfindung zugrunde liegende Aufgabe wird nun dadurch gelöst, dass die Segmente mehrere aufragende Stützelemente aufweisen, wobei der Patient zumindest teilweise von den Stützelementen abstützbar ist, wobei die Stützelemente in unterschiedlicher Höhe derart anordbar sind, so dass die Anordnung der Stützelemente der Kontur des Patienten in Längsrichtung und Querrichtung anpassbar ist, wodurch der Patient in einem mittels der Stützelemente gebildeten Abdruck gehalten ist. Dadurch, dass der Patient mit seinem Körpergewicht und entsprechend seiner Körpergröße den Liegenbereich mit den aufragenden Stützelementen individuell verformt, entsteht ein individuell gewölbter Abdruck, in dem der Patient liegt. Hierdurch ist der Patient in dem Abdruck gehalten. Dies ist für den Patienten angenehmer. Durch die Stützelemente ist der Patient fixiert. Dadurch dass die Segmente in Längsrichtung verschiebbar sind, ist der Abdruck teilbar, wodurch die Zugkräfte auf den Patienten übertragen werden. Der Abdruck ist an verschiedenen Trennstellen teilbar. Das Angebotsspektrum ist dadurch erweitert, da insbesondere der Brustwirbelbereich an jedem Wirbelgelenkpaar therapierbar ist. Der Abdruck kann bspw. eine Tiefe von mehr als 2 cm oder von mehr als 5 cm, vorzugsweise von mehr als 10 cm, insbesondere ca 15cm aufweisen. Die Tiefe des Abdrucks kann insbesondere ca. die Hälfte der Körpertiefe in der sagitalen Ebene betragen. Die Stützelemente sind vorzugsweise mehr als 5 cm, insbesondere im Wesentlichen 10 cm in der Höhe verfahrbar. Es sind vorzugsweise mehr als 10 Stützelemente, insbesondere mehr als 50 Stützelemente, vorzugsweise mehr als 100 Stützelemente vorgesehen, um eine möglichst genauen Abdruck des Körpers bilden zu können. Die Stützelemente können insbesondere weniger als 10 cm, vorzugsweise weniger als 5 cm beabstandet sein.

Insbesondere ist der Liegenbereich in einer Vielzahl von Segmenten der Länge nach aufgeteilt. Es können bspw. mehr als 10 Segmente, vorzugsweise mehr als 20 Segmente, insbesondere 30 Segmente vorgesehen sein. Die menschliche Wirbelsäule weist 24 Wirbelkörper auf. Vorzugsweise sind mehr als 24 Segmente vorgesehen in Längsrichtung hintereinander angeordnet. Jedem Wirbelkörper können ein oder zwei Segmente zugeordnet sein. Jedes der Segmente weist mehrere Stützelemente auf. Die Segmente sind in Längsrichtung der Liege nebeneinander geschichtet angeordnet. Die Segmente sind insbesondere einzeln und/oder in Gruppen in Längsrichtung verschieblich angeordnet. Jedes der Segmente weist in Querrichtung mehrere Stützelemente auf. Jedes Segment kann vorzugsweise mindestens 5, insbesondere mehr als 10 Stützelemente aufweisen.

Die Stützelemente sind in Art eines in Längsrichtung teilbaren und verschiebbaren Rasters angeordnet. Das Raster kann bspw. eine Länge von mindestens 50 cm, insbesondere von ca. 100 cm aufweisen. Das Raster kann eine Breite von mindestens 30 cm, insbesondere von ca. 50 cm aufweisen. In Längsrichtung ist das Raster durch die benachbarten Segmente eingeteilt. Die Segmente erstrecken sich vorzugsweise im Wesentlichen über die Hälfte der Breite des Liegenbereiches. Die Segmente können im Bereich der Halswirbelsäule schmaler ausgebildet sein als im Bereich der Lendenwirbelsäule. Die Breite (in Längsrichtung des Liegenbereiches gemessen) der Segmente entspricht vorzugsweise im Wesentlichen der Breite (in Längsrichtung des Liegenbereiches gemessen) eines Wirbelkörpers. Beispielsweise ist es denkbar, Segmente mit einer Längsausdehnung bzw. Breite (in Längsrichtung des Liegenbereiches gemessen) von ca. 1 cm für den Bereich der Halswirbelsäule vorzusehen, im Bereich der Brustwirbelsäule Segmente mit einer Breite von ca. 1,5 cm und im Bereich der Lendenwirbelsäule Segmente mit einer Breite von ca. 2 - 2,5 cm einzusetzen.

Es ist denkbar, die Stützelemente zu fixieren, nachdem der Patient auf der Liege liegt und die Stützelemente den Abdruck bilden. Es ist denkbar, dass die Stützelemente pneumatisch, elektrisch, magnetisch oder durch eine Federkraft entgegen der Gewichtskraft der Patienten abgestützt oder vorspannbar sind. Dadurch, dass die Liegenoberfläche sich derart der Körperform des Patienten anpasst, und gezielt einzelne Wirbelsäulenbereiche therapiert werden können, wird der Körper des Patienten allein durch sein Körpergewicht im wesentlichen in dem entstehenden Negativabdruck der Liegenoberfläche gehalten.

Die Stützelemente sind vorzugsweise im Wesentlichen stiftförmig ausgebildet, wobei die Stützelemente jeweils in einem aufragenden Zylinder verschieblich angeordnet sind. Die Stützelemente sind insbesondere mittels eines Aktors verfahrbar. Der Aktor kann insbesondere die Stützelemente pneumatisch verfahren. Das einzelne Segment weist einen Rahmen auf, wobei der Rahmen verschiebbar angeordnet ist. Der Rahmen trägt die Stützelemente. Der Rahmen weist jeweils mindestens einen Druckluftanschluss auf, um die entsprechenden Stützelemente pneumatisch zu betätigen, so dass durch Druckbeaufschlagung der Stützelemente der Anpressdruck der Stützelemente an den Körper einstellbar ist. Die Stützelemente und die entsprechenden Zylinder können in einer flexiblen Matrix aus einem nachgiebigen Material eingebettet sein. Diese Matrix kann mehrere aufragende Zylinder aufweisen, wobei die Stützelemente entsprechend in diesen Zylindern angeordnet sind. Es ist denkbar, dass die Stützelemente ein Hub innerhalb der Matrix von mehreren Zentimetern, bspw. im Wesentlichen 10 cm, ausführen können. Wenn der Patient sich nun auf die Liege legt, so wird zunächst die Matrix ein wenig komprimiert, wobei die Stützelemente nun von unten in den Zylindern hochgefahren werden, so dass der Patient zumindest teilweise an den Stützelementen abgestützt ist. Es ist denkbar, die Stützelemente mittels Federmittel am Rahmen federnd abzustützen. Jedem Stützelement kann dabei ein Federmittel, bspw. eine Schraubenfeder oder ein Federpaket, zugeordnet sein, wobei das Stützelement funktional wirksam an dem Federmittel abgestützt ist. Durch die Kompressionsfähigkeit der Federmittel sind die Stützmittel in unterschiedlicher Höhe anordbar, so dass sich der entsprechende Abdruck des Patienten bildet, wenn der Patient sich auf den Liegenbereich legt und entsprechend einsinkt.

Der Liegenbereich ist wahlweise an unterschiedlichen Trennstellen zwischen benachbarten Segmenten in eine erste Segmentgruppe und in eine zweite Segmentgruppe teilbar ist, wobei die erste Segmentgruppe und die zweite Segmentgruppe jeweils mindestens ein Segment aufweisen, wobei ein der Trennstelle benachbartes Segment der ersten Segmentgruppe und ein der Trennstelle benachbartes Segment der zweiten Segmentgruppe mittels des Antriebssystems zur Dekompression relativ zueinander verfahrbar sind. In besonders bevorzugter Ausgestaltung sind die Segmente wahlweise mit dem Antriebssystem entsprechend verbindbar. Dies hat den Vorteil, dass es möglich ist, gezielt eine bestimmte Bandscheibe oder ein Wirbelgelenkpaar zu therapieren. Insbesondere ist es möglich, eine bestimmte Bandscheibe oder ein bestimmtes Wirbelgelenkpaar mit zwei gleichgroßen, wiederholgenau einstellbaren Kräften insbesondere unter einem optimalen und wiederholgenau einstellbaren Kraftwinkel in entgegengesetzter Richtung zu entlasten. Das Therapiefeld bzw. die Trennstelle, also die zu entlastende Bandscheibe bzw. das zu entlastende Wirbelgelenkpaar wird anhand der individuellen Patientendaten eingestellt. Die Einstellung der Extensionsliegenvorrichtung auf dem zu behandelnden Therapiebereich und damit die Trennstelle erfolgt anhand anatomischer Referenzpunkte der Wirbelsäule.

Der Liegenbereich weist vorzugsweise zwei Hälften auf, wobei jede der Hälften mehrere Segmente aufweist, die jeweils in eine entsprechende obere Segmentgruppe und eine untere Segmentgruppe aufteilbar sind, wobei jeder Reihe mindestens ein Antriebsmotor zugeordnet ist. Dadurch sind seitlich unterschiedliche Zugkräfte durch die Segmentgruppen einleitbar. Ein zu behandelndes Wirbelgelenkpaar kann seitlich entlastet werden. Dies hat den Vorteil, dass bspw. ein seitlicher Bandscheibenvorfall oder andere einseitige Krankheitsbilder - bspw. ein einseitiger Wirbelgelenkverschleiß - behandelt werden kann. Es können sämtliche seitliche Achsabweichungen der Wirbelsäule entlastet werden. Das Antriebssystem kann insbesondere vier Antriebsmotoren aufweisen, wobei jeder der Antriebsmotoren jeweils eine Segmentgruppe von Segmenten bewegen kann. Beispielsweise können zwei Antriebsmotoren die erste und zweite Segmentgruppe auf der linken Hälfte antreiben und zwei Antriebsmotoren die erste und zweite Segmentgruppe auf der rechten Hälfte antreiben. Es ist wählbar, dass eine Segmentgruppe aus mehreren und oder aus einem einzelnen Segment besteht.

Die einzelnen Segmente sind dabei jeweils vorzugsweise höhenverstellbar. Die einzelnen Segmente sind vorzugsweise zumindest teilweise stufenlos höhenverstellbar. Hierdurch ist die Wirbelsäule in eine geeignete Behandlungslage (Flexionslage) bringbar und damit der Angriffswinkel der Zugkräfte genauer einstellbar, nämlich am Scheitelpunkt der Therapieebene. Dies hat den Vorteil, dass die Segmente - zusätzlich zu dem Hub der Stützelemente - an die Form der Wirbelsäule anpassbar ist. Es ist denkbar, dass jedes Segment durch ein separates Hubglied verstellbar ist. Dies ermöglicht eine besonders genaue Höhenverstellung aller Segmente. Es ist jedoch auch denkbar, dass nur einige Segmente höhenverstellbar sind, wodurch weniger Hubglieder eingesetzt werden müssen, was Kosten spart. Die Segmente sind vorzugsweise auf einer höhenprofilierten Profilschiene abgestützt, wobei insbesondere durch Verschieben der Profilschiene unterschiedliche Stellungen der Segmente einstellbar sind. Ferner kann es möglich sein, die Profilschiene zu schwenken, um die Höhenlage der Segmente zu verändern. Dies ist eine einfach herzustellende, kostengünstige Ausgestaltung, um die Segmente in der Höhe zu verfahren und die Wirbelsäule dadurch in die gewünschte Behandlungsposition zu bringen, die angepasst ist an die individuelle Anatomie zur Erzielung eines stufenlosen, optimalen Einstellwinkels des zu entlastenden Bereiches.

Ferner ist es denkbar, zusätzliche Mittel zur Wärmebehandlung oder Elektrostimulation einzusetzen, um die oberflächliche und/oder tiefe Rückenmuskulatur des Patienten zu detoniseren. In bevorzugter Ausgestaltung wird die Wärme und/oder eine Massage über eine Vibration oder dergleichen über die Stützelemente eingebracht. Über die insbesondere elektrisch leitenden Stützelemente kann eine Gleichstrombehandlung erfolgen.

Es ist denkbar, dass zusätzliche Haltemittel zur Verbindung des Patienten mit den Segmenten vorgesehen sind. Es ist denkbar, dass zumindest einige der Segmente Ansaugöffnungen aufweisen, wobei die Ansaugöffnungen mit einem Unterdruckerzeuger verbunden oder verbindbar sind. Hierdurch ist es möglich, den Patienten mittels Unterdruck an das entsprechende Segment anzusaugen, um die entsprechenden Zugkräfte auf den Patienten zu übertragen. Ferner ist es denkbar, dass als Haltemittel Gurte vorgesehen sind, wobei der Patient mittels der Gurte mit den Segmenten verbindbar ist. Die Stützelemente und damit der Abdruck übertragen hierbei einen Teil der Zugkräfte und ein anderer Teil der Zugkräfte wird durch die zusätzlichen Haltemittel auf den Patienten übertragen. Es werden hierdurch nur geringere Kräfte durch die zusätzlichen Haltemittel übertragen, so dass die Behandlung für den Patienten immer noch angenehmer ist, als wenn die Zugkräfte ausschließlich durch Gurte übertragen würden.

Die eingangs genannten Nachteile sind daher vermieden und entsprechende Vorteile sind erzielt.

Es gibt nun eine Vielzahl von Möglichkeiten, die erfindungsgemäße Extensionsliegenvorrichtung in vorteilhafter Art und Weise auszugestalten und weiterzubilden. Hierfür darf zunächst auf die dem Patentanspruch 1 nachgeordneten Patentansprüche verwiesen werden. Im folgenden werden drei bevorzugte Ausgestaltungen der Erfindung anhand der Zeichnung und der dazugehörigen Beschreibung näher erläutert.

In der Zeichnung zeigt:
- Fig. 1: in einer schematischen, perspektivischen Darstellung eine erste Extensionsliegenvorrichtung mit einem Liegenbereich und mit einem Computer,
- Fig. 2: in einer schematischen, perspektivischen Darstellung die erste Extensionsliegenvorrichtung, wobei der Liegenbereich in einer anderen Stellung angeordnet ist,
- Fig. 3: in einer schematischen Draufsicht die erste Extensionsliegenvorrichtung,
- Fig. 4: in einer schematischen Seitenansicht die erste Extensionsliegenvorrichtung,
- Fig. 5: in einer schematischen, seitlichen Detaildarstellung den Liegenbereich mit einem Teil eines Antriebsystems,
- Fig. 6: in einer seitlichen Detaildarstellung den Liegenbereich mit dem Teil des Antriebsystems, wobei der Liegenbereich in einer anderen Stellung angeordnet ist,
- Fig. 7: in einer schematischen, perspektivischen Detaildarstellung mehrere nebeneinander angeordnet Segmente, die Teil des Liegenbereiches sind,
- Fig. 8: in einer schematischen, geschnittenen Darstellung eines der Segmente im Schnitt,
- Fig. 9: in einer schematischen, perspektivischen Darstellung die erste Extensionsliegenvorrichtung mit einem stark schematisch dargestellten Patienten, wobei der Liegenbereich in einer aufragenden Einstiegsposition zum Sitzen und Einstieg des Patienten angeordnet ist,
- Fig. 10: in einer schematischen Seitenansicht die Extensionsliegenvorrichtung, wobei der Patient auf dem Liegenbereich liegt,
- Fig. 11: in einer schematischen Seitenansicht die Extensionsliegenvorrichtung, wobei der Patient mit angewinkelten Beinen auf dem eingestellten Liegenbereich liegt,
- Fig. 12a: in einer schematischen Seitenansicht die erste Extensionsliegenvorrichtung, wobei der Patient zur Therapie der Brustwirbelsäule mit angewinkelten Beinen in einer weiteren Stellung des Liegenbereiches auf dem Liegenbereich liegt,
- Fig. 12b: in einer schematischen Seitenansicht die erste Extensionsliegenvorrichtung, wobei der Patient zur Therapie der Halswirbelsäule mit angewinkelten Beinen in einer weiteren Stellung des Liegenbereiches auf dem Liegenbereich liegt,
- Fig. 13: in einer schematischen, perspektivischen Darstellung eine zweite Extensionsliegenvorrichtung,
- Fig. 14: in einer schematischen Darstellung die zweite Extensionsliegenvorrichtung, wobei der Liegenbereich in einer aufragenden Einstiegsposition zum stehenden Einstieg eines nicht dargestellten Patienten angeordnet ist,
- Fig. 15: in einer schematischen, perspektivischen Detaildarstellung ein Teil des Liegenbereiches der zweiten Extensionsliegenvorrichtung,
- Fig. 16: in einer schematischen, perspektivischen Detaildarstellung, den Liegenbereich schräg von unten mit einem Teil eines Antriebsystems,
- Fig. 17: in einer schematischen, perspektivischen Darstellung der Liegenbereich schräg von oben,
- Fig. 18: in einer schematischen, seitlichen Detailansicht den Liegenbereich mit einem Teil des Antriebsystems,
- Fig. 19: in einer schematischen, perspektivischen Darstellung die zweite Extensionsliegenvorrichtung, wobei ein Patient ausgestreckt auf dem Liegenbereich liegt
- Fig. 20: in einer schematischen, perspektivischen Darstellung die zweite Extensionsliegenvorrichtung, wobei der Patient in einer leicht angewinkelten Stellung auf dem Liegenbereich liegt,
- Fig. 21: in einer stark schematischen Darstellung ein Wirbelsäulenmodel, das auf mehreren Segmenten mit entsprechenden Stützelementen abgestützt ist,
- Fig. 22: in einer schematischen Draufsicht eine dritte Extensionsliegenvorrichtung,
- Fig. 23: in einer schematischen Seitenansicht die dritte Extensionsliegenvorrichtung mit einem schematisch als Skelett dargestelltem Patienten,
- Fig. 24: in einer schematischen, geschnittenen Seitenansicht die dritte Extensionsliegenvorrichtung, und
- Fig. 25: in einer schematischen, geschnittenen, perspektivischen Detaildarstellung die dritte Extensionsliegenvorrichtung.

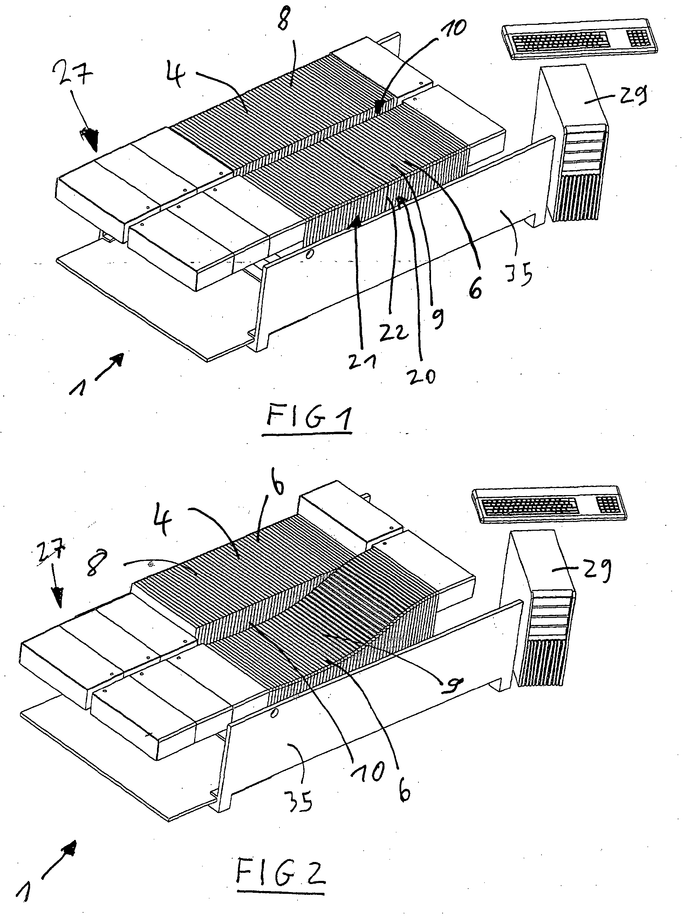

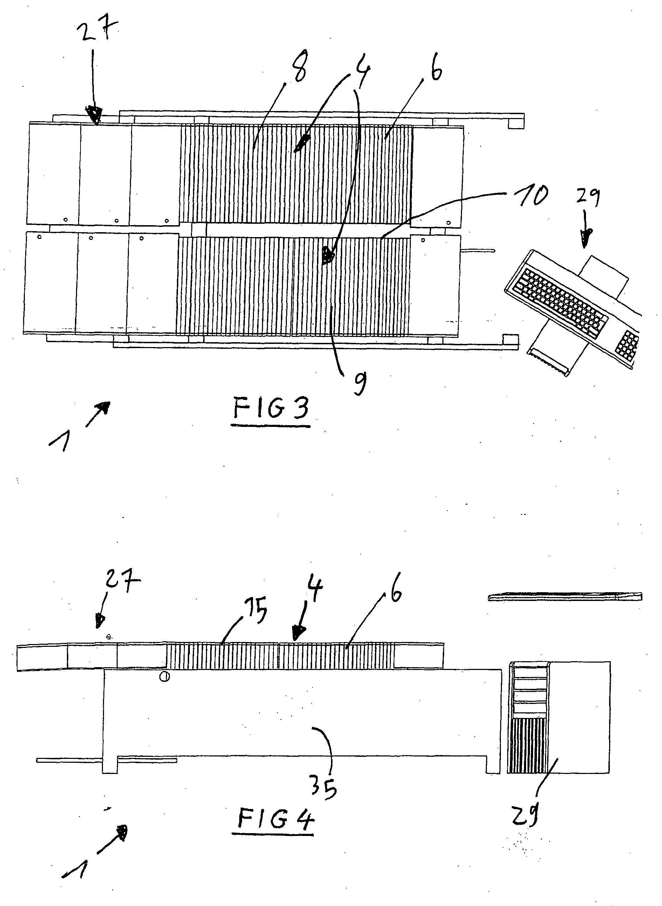

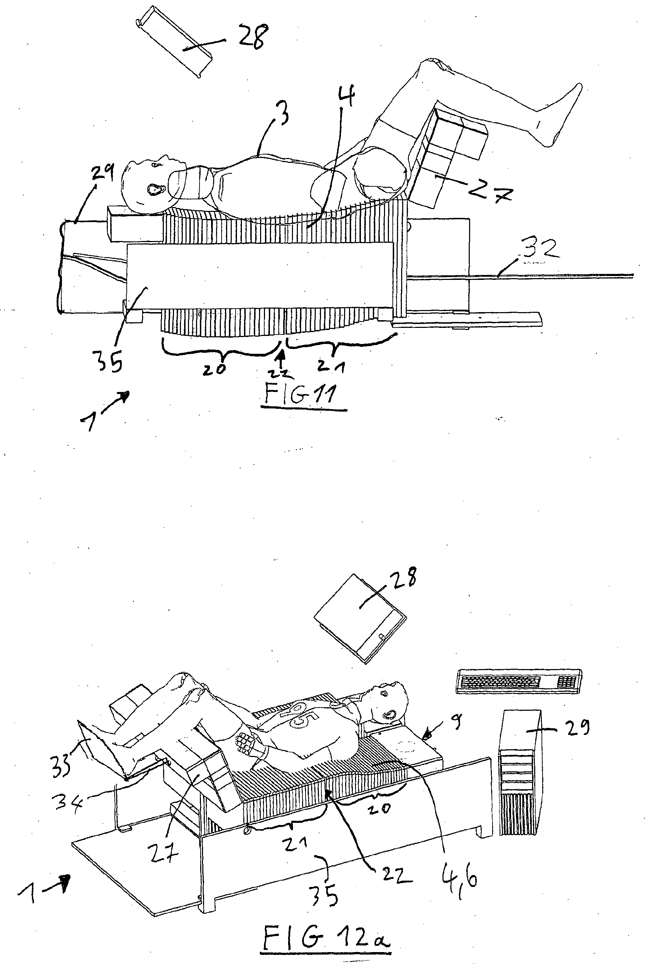

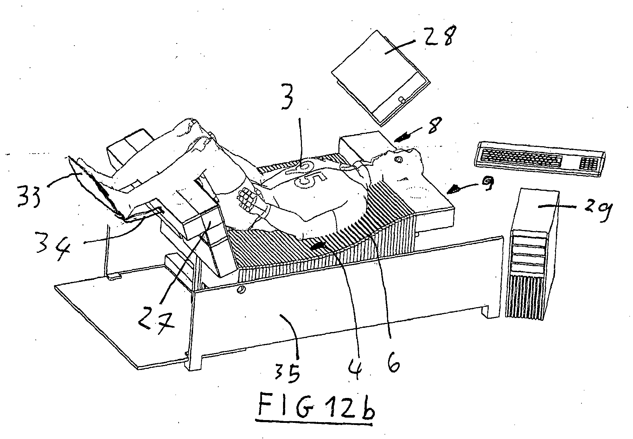

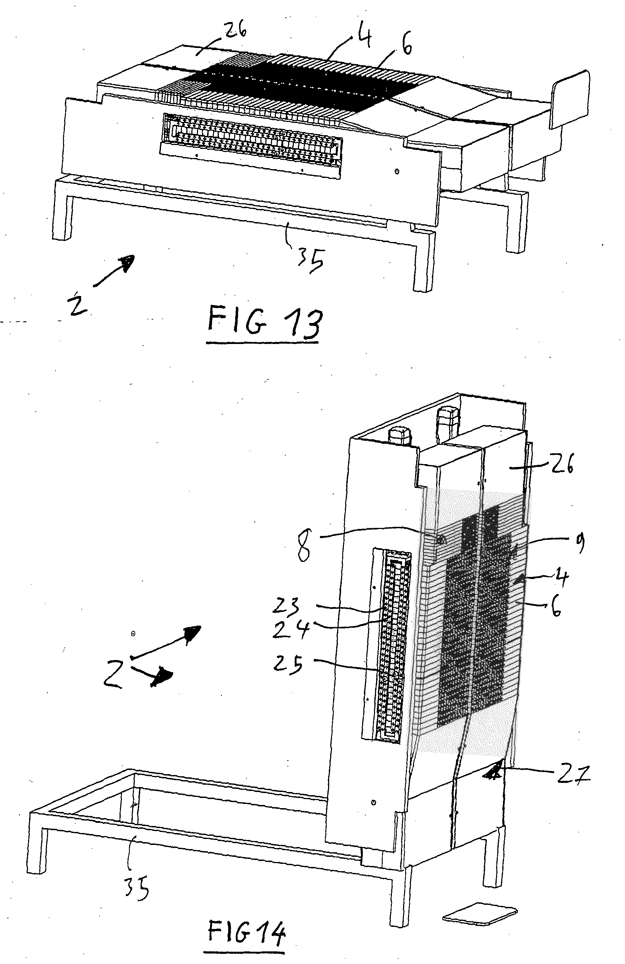

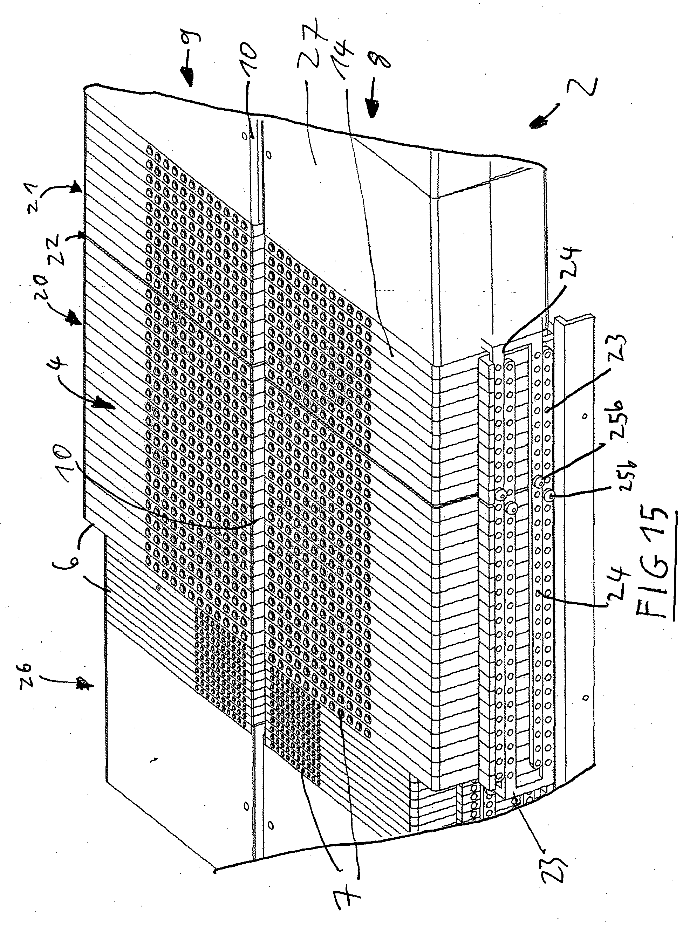

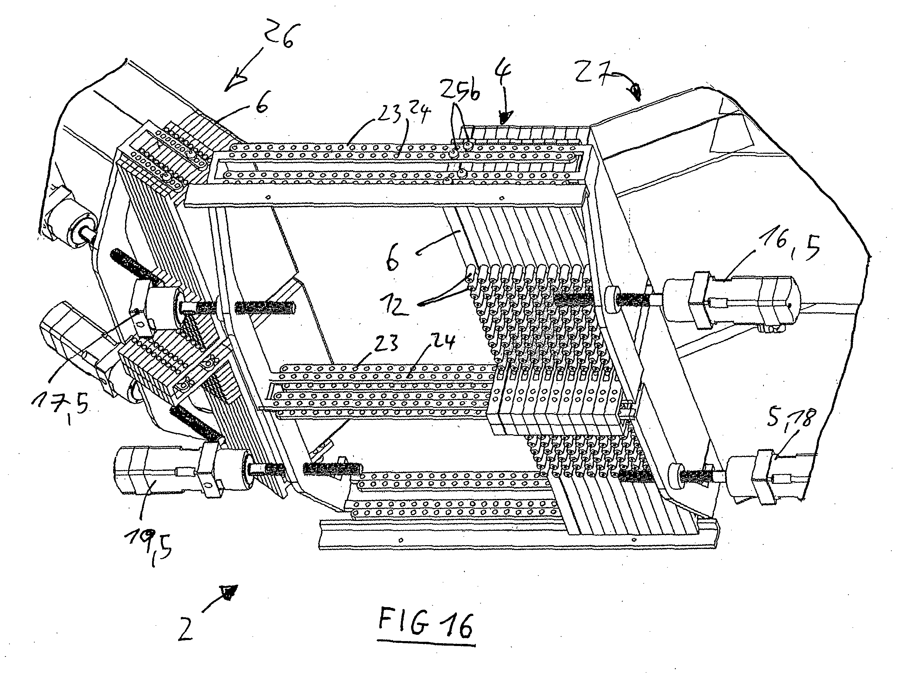

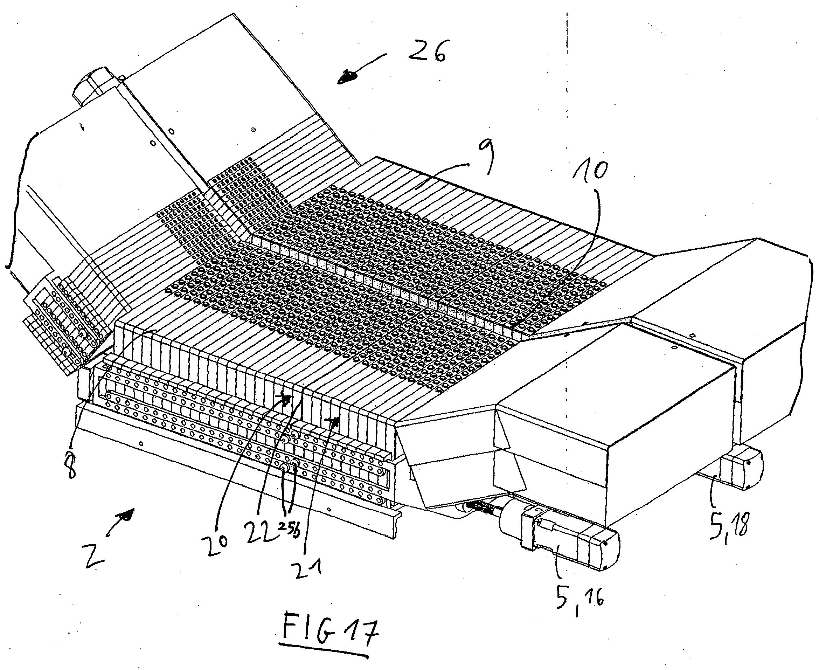

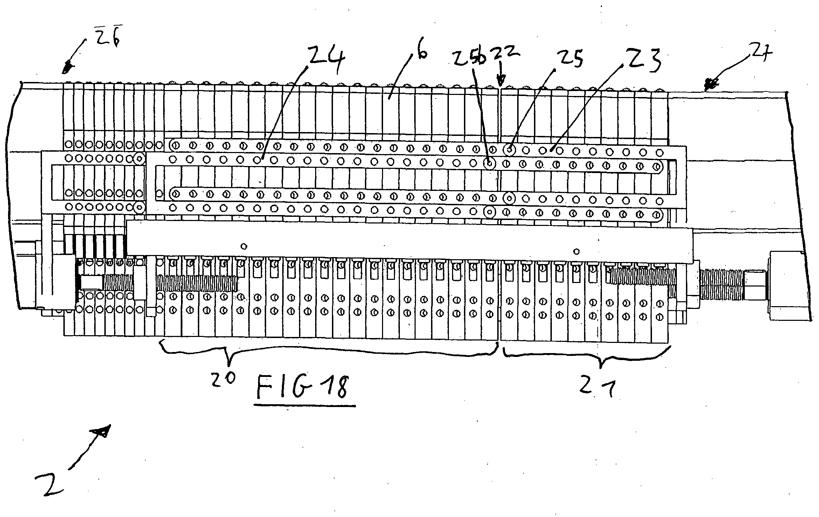

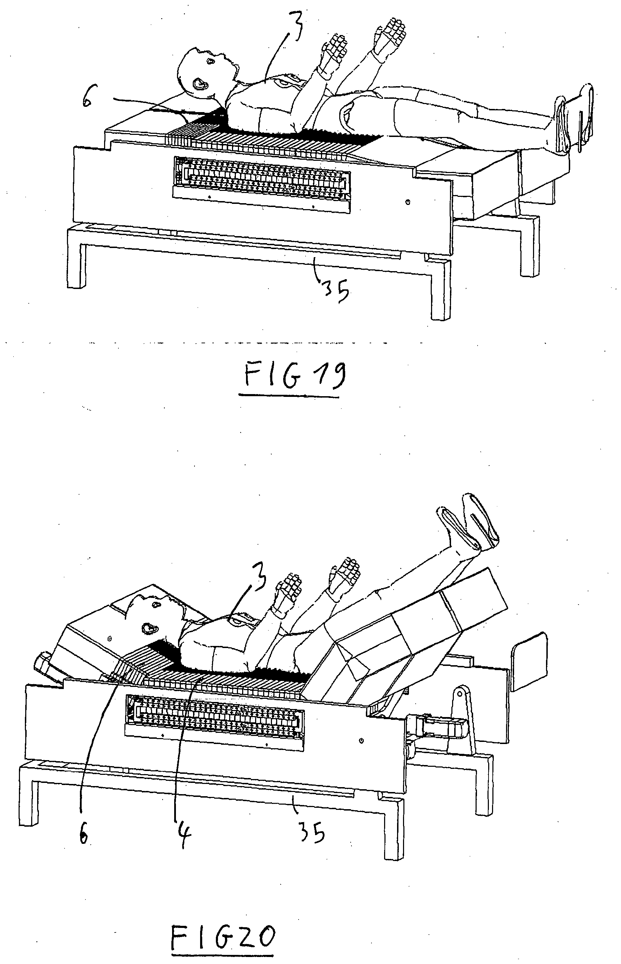

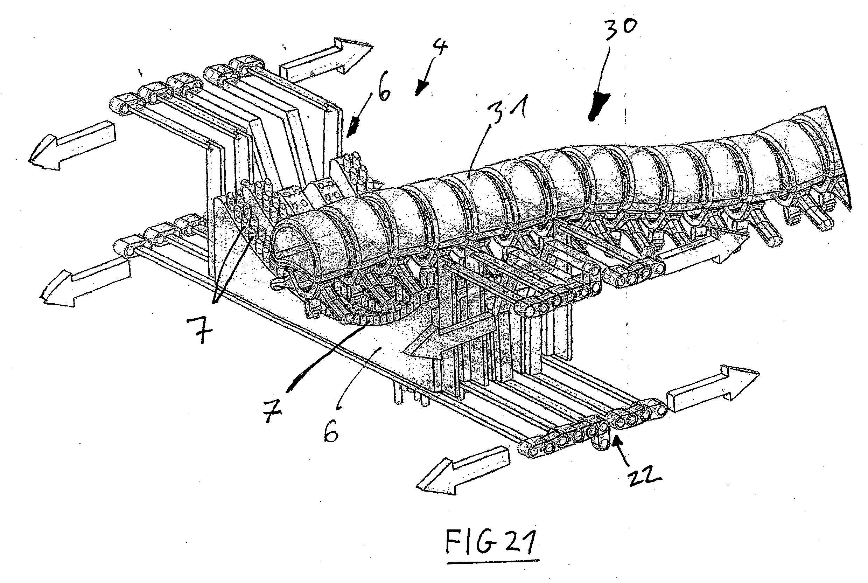

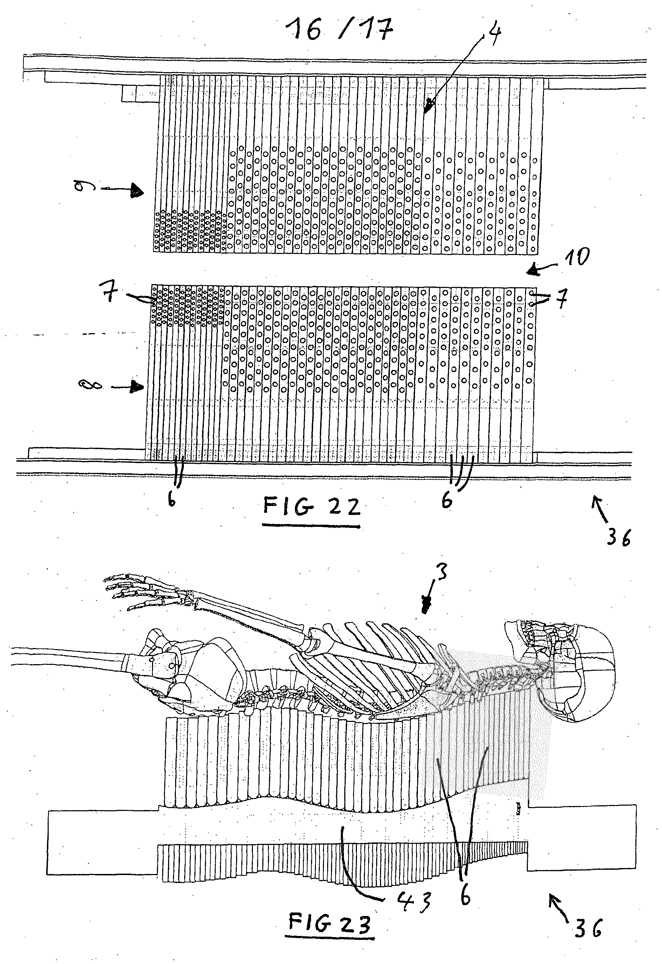

In den Fig. 1 bis 12 ist eine erste Extensionsliegenvorrichtung 1, in den Fig. 13 bis 20 ist eine zweite Extensionsliegenvorrichtung 2 und in Fig. 22 bis 25 ist eine dritte Extensionsliegenvorrichtung 36 zu erkennen. Die Extensionsliegenvorrichtungen 1, 2 und 36 dienen zur Dekompression der Wirbelsäule 30 eines Patienten 3 (vgl. Fig. 21, 23). Die Erfindung ist jedoch auch zur Dekompression von anderen Gelenken, bspw. den Gelenken der Extremitäten einsetzbar.

Im folgenden dürfen zunächst die Gemeinsamkeiten der Extensionsliegenvorrichtungen 1 und 2 sowie 36 beschrieben werden, wobei für im Wesentlichen gleichartige Bauteile oder Baugruppen gleiche Bezugszeichen verwendet werden.

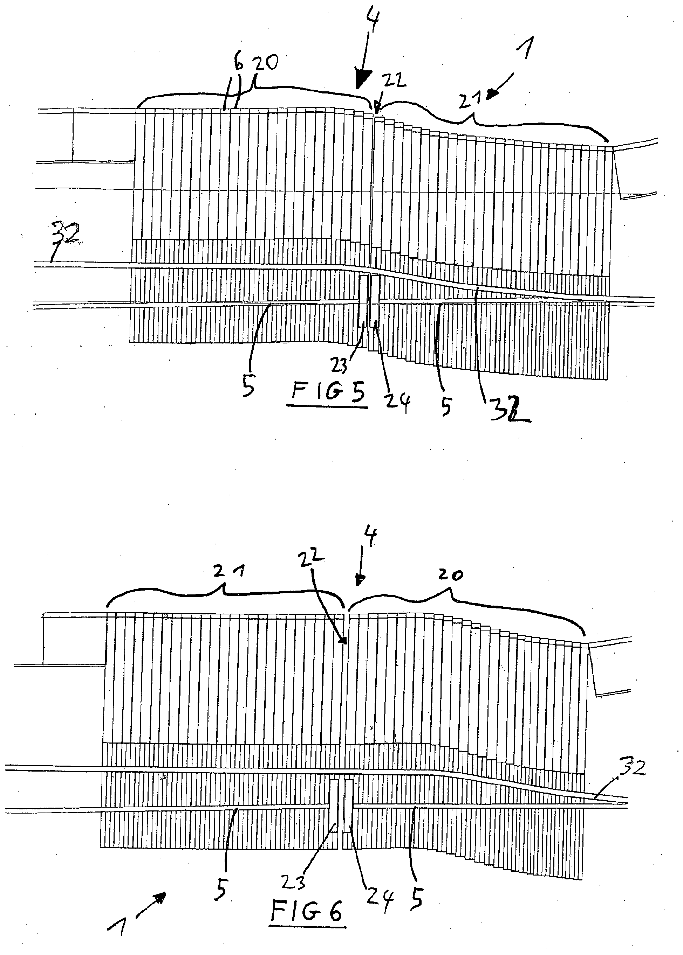

Die Extensionsliegenvorrichtungen 1, 2 und 36 weisen einen Liegenbereich 4 und ein Antriebsystem 5 (vgl. Fig. 5 und 6) auf. Der Liegenbereich 4 weist mehrere Segmente 6 auf. Die Segmente 6 sind mit dem Antriebsystem 5 derart bewegbar, so dass eine Zugkraft und eine Gegenzugkraft auf den Patienten 3 ausübbar ist, während der Patient 3 zur Dekompression der Wirbelsäule 30 auf dem Liegenbereich 4 liegt. Das Funktionsprinzip ist beispielhaft in Fig. 21 dargestellt.

Die eingangs genannten Nachteile sind nun dadurch vermieden, dass die Segmente 6 mehrere aufragende Stützelemente 7 aufweisen, wobei der Patient 3 zumindest teilweise von den Stützelementen 7 abstützbar ist, wobei die Stützelemente 7 in unterschiedlicher Höhe derart anordbar sind, so dass die Anordnung der Stützelemente 7 der Kontur des Patienten 3 in Längsrichtung und Querrichtung anpassbar ist, wodurch der Patient 3 in einem mittels der Stützelemente 7 gebildeten Abdruck gehalten ist. Dieser (Negativ-)Abdruck entsteht durch unterschiedlich starkes Einsinken des Patienten 3 in die Liegenoberfläche, nämlich in Abhängigkeit des Körpergewichtes des Patienten 3. Dadurch dass der Patient 3 in dem Abdruck liegt, ist er mit den Segmenten 6 zumindest teilweise formschlüssig verbunden.

Jedes der Segmente 6 weist insbesondere eine Vielzahl von Stützelementen 7 auf. Es können bspw. insgesamt mehr als 100 Stützelemente 7 vorgesehen sein, damit diese die Stellung einer Negativform des Patienten 3 einnehmen können. Legt sich nun ein Patient 3 auf den Liegenbereich 4, so werden die Stützelemente 7 in Abhängigkeit der einwirkenden Gewichtskraft unterschiedlich stark nach unten verschoben.

In Fig. 21 ist das Modell einer Wirbelsäule 30 mit mehreren Wirbelkörpern 31 dargestellt, wobei die Wirbelsäule 30 auf zwei Segmenten 6 mit jeweils einer Vielzahl von stiftförmigen Stützelementen 7 abgestützt ist. Zwischen den beiden Segmenten 6 ist eine Trennstelle 22 vorgesehen, so dass die Segmente 6 auseinanderziehbar sind und damit die entsprechenden, aufliegenden Wirbelkörper 31 dekompressierbar sind.

Die Anordnung der Stützelemente 7 bildet ein Raster, wobei durch die einwirkende Gewichtskraft des Patienten 3 dieses Raster bestehend aus den Stützelementen 7 einen Negativabdruck der Körperform des Patienten 3 abbildet. Durch diesen Negativabdruck wird der Patient 3 zumindest teilweise fixiert, so dass mit dem Antriebsystem 5, insbesondere zwei Segmente 6 derart auseinander gezogen werden können, so dass eine Dekompression der aufliegenden Wirbelsäule 30 stattfindet. Durch den Negativabdruck ist es vorzugsweise möglich, auf ein Gurtsystem zu verzichten, das den Patienten 3 fixiert. Es ist jedoch denkbar, unterstützende Halterungen vorzusehen, um den Patienten 3 in dem Abdruck zu halten. Die Extensionsliegenvorrichtung 1, 2 kann aufblasbare Kammern (nicht dargestellt) insbesondere seitlich des Patienten 3 aufweisen, so dass der Patient 3 durch Aufblasen der Kammern besonders gut in dem entstehenden Abdruck gehalten wird. Die Kammern können sich insbesondere seitlich des Patienten 3 erstrecken und im aufgeblasenen Zustand zusätzliche Seitenwände des Abdruckes bilden. Diese Kammern sind mit mindestens einer Pumpe verbunden, so dass ein entsprechendes Fluid, insbesondere Luft, in die Kammern pumpbar ist. Das Fluid kann nach dem Ende der Therapie aus den Kammern wieder ausgelassen werden, bspw. durch Abpumpen des Fluids.

Es ist denkbar weitere Halterungen oder Haltemittel vorzusehen, um die Zugkrafteinleitung über den Abdruck zusätzlich zu unterstützen. Es ist denkbar, dass zumindest einige der Segmente 6 Ansaugöffnungen aufweisen, wobei die Ansaugöffnungen mit einem Unterdruckerzeuger verbunden oder verbindbar sind (nicht dargestellt). Hierdurch ist es möglich, den Patienten mittels Unterdruck an das entsprechende Segment 6 anzusaugen, um die entsprechenden Zugkräfte auf den Patienten 3 zu übertragen. Ferner ist es denkbar, dass als Haltemittel Gurte vorgesehen sind, wobei der Patient 3 mittels der Gurte mit den Segmenten 6 verbindbar ist (nicht dargestellt), um einen Teil der Zugkräfte über die Gurte zu übertragen.

Der Liegenbereich 4 ist vorzugsweise in zwei Hälften 8, 9 mit jeweils mehreren Segmenten 6 aufgeteilt. Die Hälften 8,9 erstrecken sich in Längsrichtung parallel zueinander und weisen vorzugsweise die gleiche Anzahl an Segmenten 6 auf. Zwischen den beiden Hälften 8, 9 ist ein Spalt 10 ausgebildet. Die beiden Hälften 8, 9 bilden jeweils die linke und die rechte Hälfte (nicht näher bezeichnet) des Liegenbereichs 4. Jede Hälfte 8, 9 weist eine Reihe von Segmenten 6, insbesondere mehr als drei Segmente 6, insbesondere mehr als zehn Segmente 6, vorzugsweise mehr als 20 Segmente 6, insbesondere 30 Segmente 6 auf. Hierdurch ist der Liegenbereich 4 in eine Vielzahl von Segmenten 6 der Länge nach aufgeteilt. Die Segmente 6 können im Bereich der Halswirbelsäule eine geringere Längsausdehnung in Längsrichtung des Liegenbereiches bzw. Breite aufweisen als im Bereich der Lendenwirbelsäule. Beispielsweise ist es denkbar, Segmente 6 mit einer Breite von ca. 1 cm für den Bereich der Halswirbelsäule einzusetzen, im Bereich der Brustwirbelsäule Segmente 6 mit einer Breite von ca. 1,5 cm und im Bereich der Lendenwirbelsäule Segmente 6 mit einer Breite von ca. 2 bis 2,5 cm. Die Segmente 6 können in ihrer Breite vom Becken zum Kopf in der Breite (in Längsrichtung des Liegenbereiches 4 gemessen) abnehmen.

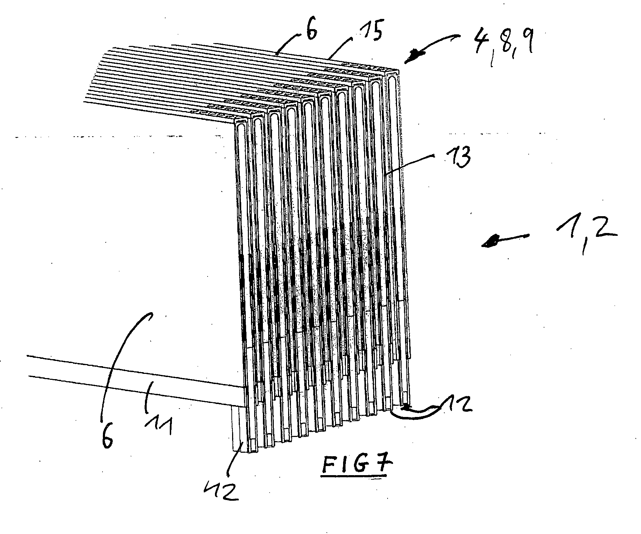

Jedes der Segmente 6 weist dabei im Wesentlichen in Querrichtung nebeneinander angeordnete, aufragende stiftförmige Stützelemente 7 auf. In Fig. 7 ist ein Längsschnitt durch einen Teil einer Reihe 8, 9 von mehreren nebeneinander aufragenden Segmenten 6 dargestellt.

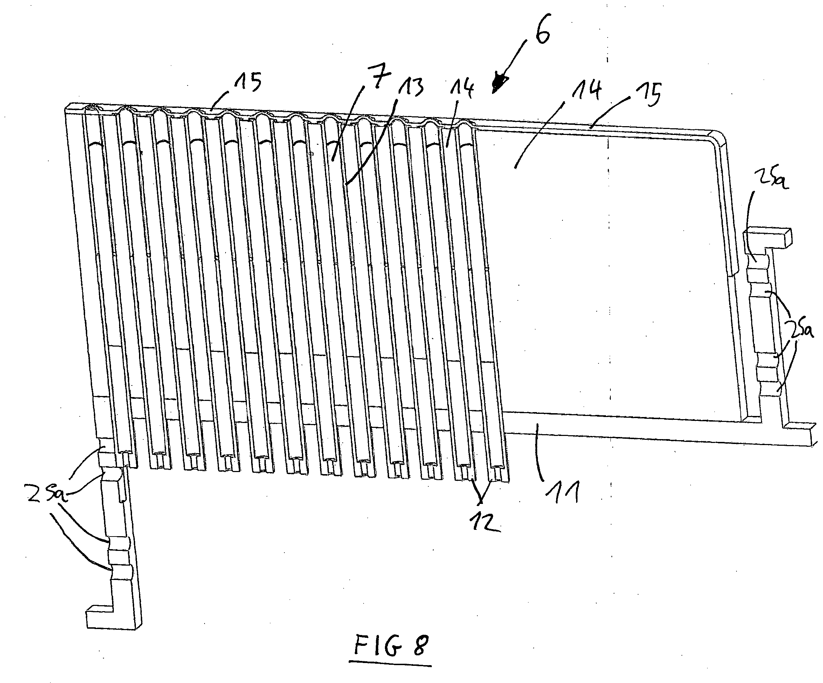

Es darf im folgenden auf Fig. 8 eingegangen werden. Die Stützelemente 7 sind insbesondere pneumatisch oder hydraulisch mittels mindestens eines Aktors (nicht dargestellt) verfahrbar. In alternativer Ausgestaltung ist es denkbar, die Stützelemente 7 hydraulisch oder elektromotorisch zu verfahren. Das dargestellte Segment 6 weist einen Rahmen 11 auf. Der Rahmen 11 weist mehrere Druckluftanschlüsse 12 auf. In der dargestellten Ausgestaltung ist hier jedem der Druckluftanschlüsse 12 eines der Stützelemente 7 zugeordnet. Es sind jedoch auch Ausgestaltungen möglich, wobei mehreren Stützelementen 7 ein einziger Druckluftanschluss 12 zugeordnet ist. Die Stützelemente 7 sind in Zylindern 13 verschiebbar angeordnet. Jeder der Zylinder 13 steht funktional wirksam mit zumindest einem der Druckluftanschlüsse 12 in Verbindung. Die Zylinder 13 sind hier in einer Matrix 14 eingebettet. Die Matrix 14 kann bspw. im Wesentlichen aus einem Schaumstoff oder dergleichen bestehen. Die Matrix 14 ist nachgiebig ausgebildet. Zwischen den einzelnen Zylindern 13 erstreckt sich die Matrix 14. Die Matrix 14 liegt jeweils auf dem Rahmen 11 funktional wirksam auf. Die Matrix 14 kann links und rechts, seitlich der Stützelemtanordnung die oben genannten Kammern aufweisen, um so zusätzliche Seitenwände des Abdruckes zu bilden (nicht dargestellt). Die Stützelemente 7 können insbesondere weniger als 5 cm beabstandet sein. Der Härtegrad der Liegenoberfläche kann in den einzelnen Segmenten 6 unterschiedlich sein. Die Oberfläche des Liegenbereiches 4 ist insbesondere durch eine Auflage 15 abgedeckt. Die Auflage 15 ist abwischbar. Die Auflage 15 ist insbesondere desinfektionsfest ausgebildet. Die Auflage 15 deckt die einzelnen Zylinder 13 ab. Die Auflage 15 ist dehnbar ausgebildet. Wenn nun die Stützelemente 7 nach oben verfahren werden, so beulen die Stützelemente 7 die Auflage 15 an den entsprechenden Stellen aus, so dass der Patient 3 auf den gewölbten Stellen, d.h. den darunter aufragenden Stützelementen 7 abgestützt ist.

In Fig. 22 ist erkennbar, dass in bevorzugter Ausgestaltung die Stützelement 7 benachbarter Segmente 6 in Längsrichtung 6 gesehen seitlich versetzt angeordnet sind, um einen möglichst guten Halt zu vermitteln. Durch die versetzte Anordnung wird vermieden, dass die Stützelemente 7 benachbarter Segmente 6 jeweils schon in "eingedellte Bereiche" des Rückens des Patienten 3 eingreifen. Durch die versetzte Anordnung ist in Längsrichtung und damit in Zugrichtung ein möglichst großer Bereich des Rückens des Patienten 3 mittels der Stützelemente 7 greifbar.

Wenn sich nun der Patient 3 auf den Liegenbereich 4 legt, so sinkt der Patient 3 nun zumindest teilweise in den Liegenbereich 4 ein. Werden nun die Stützelemente 7 derart nach oben verfahren, so dass sie den Patienten 3 zumindest teilweise abstützen, so bildet sich der beschriebene (Negativ-)Abdruck der Kontur des Patienten 3. Die Stützelemente 7 werden durch Anlegen der entsprechenden Druckluft verfahren. Es ist denkbar, dabei je nach Bereich, bspw. Halswirbelbereich, Brustwirbelbereich oder Lendenwirbelbereich unterschiedliche Gegenkräfte durch die Stützelemente 7 bereitzustellen und/oder eine unterschiedliche Anzahl von Stützelementen 7 in den entsprechenden Bereich vorzusehen.

Es ist alternativ denkbar, die Stützelemente 7 mittels entsprechender Federmittel abzustützen (nicht näher dargestellt). Dies ist kostengünstiger als wenn die Stützelemente 7 mittels entsprechender Aktoren einzeln verfahrbar sind. Durch das Gewicht des Patienten 3 werden die Federmittel komprimiert, wodurch der Patient in den Liegenbereich 4 einsinkt und der entsprechende Abdruck gebildet wird.

Die Segmente 6 sind mittels des Antriebssystems 5 wahlweise derart in Längsrichtung verfahrbar, so dass unterschiedliche Rückenbereiche des Patienten 3 therapiert werden können. Insbesondere sind die Segmente 6 wahlweise mit dem Antriebssystem 5 verbindbar.

Der Liegenbereich 4 ist wahlweise an unterschiedlichen Trennstellen 22 zwischen benachbarten Segmenten 6 in eine erste Segmentgruppe 20 und in eine zweite Segmentgruppe 21 teilbar, wobei die erste Segmentgruppe 20 und die zweite Segmentgruppe 21 jeweils mindestens ein Segment 6 aufweisen, wobei ein der Trennstelle 22 benachbartes Segment 6 der ersten Segmentgruppe 20 und ein der Trennstelle 22 benachbartes Segment 6 der zweiten Segmentgruppe 21 mittels des Antriebsystems 5 zur Dekompression relativ zueinander verfahrbar sind. Zu jeder eingeleiteten Zugkraft durch Bewegen der oberen Segmentgruppe 20 bzw. der unteren Segmentgruppe 21 wird eine Gegenzugkraft in entgegengesetzter Richtung durch die entsprechend andere Segmentgruppe 21 bzw. 20 eingeleitet.

Das Therapiefeld und damit eine virtuelle Trennstelle 22 werden anhand der individuellen Patientendaten eingestellt. Die Einstellung der Extensionsliegenvorrichtung 1, 2 auf den zu behandelnden Therapiebereiche und damit die Trennstelle 22 erfolgt anhand anatomischer Referenzpunkte der Wirbelsäule 30. Diese anatomischen Referenzpunkte können bspw. mit einer Messsonde gemessen werden, die die Wirbelsäule 30 des Patienten 3 abtastet, während der Patient 3 auf dem Liegenbereich 4 liegt. Die Messsonde kann entlang des Spaltes 10 verfahren werden und dabei die Wirbelsäule 30 abtasten.

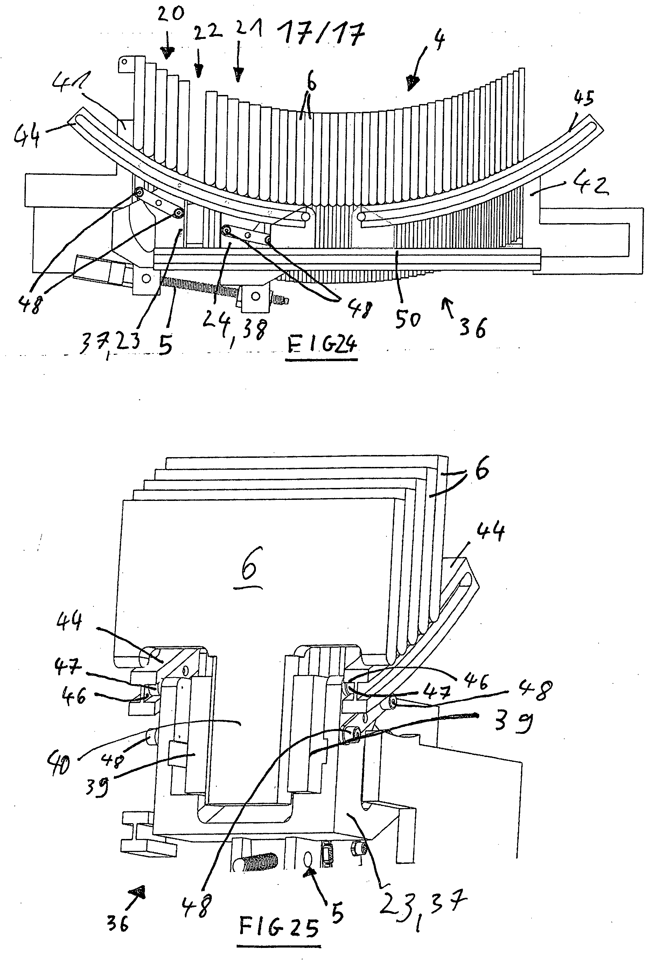

Das Antriebssystem 5 weist vorzugsweise mindestens zwei Antriebsmotoren, insbesondere vier Antriebsmotoren 16, 17, 18, 19 auf, um die entsprechenden Segmentgruppen 20, 21 in entgegensetzte Richtung zu verschieben. Es sind insbesondere zwei obere Antriebsmotoren 17 und 19 und zwei untere Antriebsmotoren 16, 18 vorgesehen. Die beiden Antriebsmotoren 16 und 17 sind dabei der linken Reihe 8 und die beiden Antriebsmotoren 18 und 19 sind der rechten Reihe 9 zugeordnet. Es ist denkbar, dass eine Segmentgruppe 20, 21 jeweils aus einem oder aus mehreren Segmenten 6 besteht. Jede Reihe 8, 9 ist in eine erste Segmentgruppe 20 und in eine zweite Segmentgruppe 21 durch entsprechende Kopplung des Antriebssystems 5 einteilbar. Zwischen den beiden Segmentgruppen 20, 21 ist eine virtuelle Trennstelle 22 gebildet. Zwischen der ersten Segmentgruppe 20 und der zweiten Segmentgruppe 21 bildet sich nun ein Spalt, wenn mit dem Antriebssystem 5 die erste Segmentgruppe 20 und die zweite Segmentgruppe 21 entsprechend zur Dekompression auseinander gezogen werden (vgl. Fig. 24). Es ist denkbar, dass das Antriebssystem 5 pneumatische, hydraulische oder elektromotorisch ausgebildet ist.

Das Antriebssystem 5 weist vorzugsweise jeweils mindestens ein erstes Koppelmittel 23 und ein zweites Koppelmittel 24 auf, wobei das erste Koppelmittel 23 zur Bewegung der zugeordneten, ersten Segmentgruppe 20 und das zweite Koppelmittel 24 zur Bewegung der zweiten Segmentgruppe 21 dient. Die Koppelmittel 23, 24 sind entsprechend mit den oberen Antriebsmotoren 17, 19 bzw. den unteren Antriebsmotoren 16, 18 verfahrbar. Die Koppelmittel 23, 24 können als Koppelstangen ausgebildet sein (vgl. Fig. 14 bis 18). Alternativ können die Koppelmittel 23, 24 als Zugseile, Drähte, Ketten od. dgl. gebildet sein.

In der in Fig. 23 bis 25 dargestellten Ausgestaltung sind die Koppelmittel 23 als in Längsrichtung verfahrbare Schlitten 37, 38 ausgebildet. Diese Schlitten 37, 38 sind mittels des Antriebssystems 5 in Längsrichtung verfahrbar. Jeder Schlitten 37, 38 weist einen seitlichen Greifer 39 auf. Die Segmente weisen dabei einen zwischen die beiden fluchtend zueinander angeordneten Greifer 39 reichenden Greifbereich 40 (vgl. Fig. 25) auf. Die Greifer 39 können mittels entsprechender Servos (nicht dargestellt) oder pneumatisch, oder hydraulisch aufeinander zu und voneinander weg bewegt werden, so dass die der Trennstelle 22 benachbarten Segmente 6 entsprechend lösbar mit den Greifern 39 und damit mit den Schlitten 37, 38 verbindbar sind. Durch Verfahren der Schlitten 37, 38 können die der Trennstelle 22 benachbarten Segmente 6 auseinander bewegt werden. In Fig. 24 sind ferner Gegenhaltplatten 41, 42 dargestellt, die dazu dienen, die Segmentgruppen 20, 21 zusammenzuhalten.

Dadurch, dass insbesondere zwischen allen benachbarten Segmenten 6 die Trennstelle 22 realisiert werden kann, d. h. dadurch dass der Liegenbereich 4 derart in eine "obere", erste Segmentgruppe 20 und eine "untere", zweite Segmentgruppe 21 eingeteilt werden kann, kann jede beliebige Bandscheibe bzw. jedes beliebige Wirbelgelenkpaar bzw. der Bandscheibe gezielt und einzeln therapiert werden. Hierdurch ist eine lokale Therapie des entsprechenden Wirbelgelenkpaares realisierbar. Die Angriffszugpunkte beim Auseinanderziehen bzw. bei der Dekompression der Wirbelsäule 30 sind hier durch die Wahl der Trennstelle 22 im Wesentlichen frei wählbar. Durch die Aufteilung des Liegenbereiches 4 in eine Vielzahl von in Längsrichtung benachbarter Segmente 6 ist das Therapiefeld und die Trennstelle 22 entsprechend fein einstellbar.

Die Koppelmittel 23, 24 können mit den einzelnen Segmenten 6 bspw. durch einen Bolzen 25b fixiert werden (vgl. Fig 14 bis 18). Dazu weist der Rahmen 11 insbesondere mindestens eine entsprechende Bolzenaufnahme 25a zur Fixierung der Bolzen 25b auf. Dadurch, dass der Liegenbereich 4 in der Mitte zweigeteilt ist, kann jede der Hälften 8, 9 mit einer Kraft und einer Gegenkraft angesteuert werden. Hierdurch ist es möglich, dass bspw. nur eine Hälfte 8 mit einer entsprechenden oberen Segmentgruppe 20 und einer entsprechenden unteren Segmentgruppe 21 auseinandergezogen wird. Bandscheibenvorfälle können bspw. auch nur einseitig auftreten, so dass es sinnvoll sein kann, entsprechend nur eine Dekompression linksseitig oder rechtsseitig der Wirbelsäule 30 mit der entsprechende Hälfte 8, 9 vorzunehmen. Dadurch können auch resultierende Zugkräfte eingeleitet werden, die tangential zu einer seitwärts gekrümmten Wirbelsäule verlaufen. Insbesondere sind die Zugkräfte dabei tangential zum Scheitelpunkt der Krümmung. Die Trennstelle 22 liegt dabei am Scheitelpunkt der Krümmung. Durch die Aufteilung in zwei Hälften 8, 9 kann die Zugrichtung eingestellt werden. Es ist bspw. denkbar, durch das Anlegen entsprechender Zugrichtungen eine Skoliose zu behandeln.

Ferner weisen die Extensionsliegenvorrichtungen 1, 2 einen schwenkbaren Beinablagebereich 27 auf (vgl. Fig. 1 bis 20). (Der Beinablagebereich ist für die Extensionsliegenvorrichtung 36 ebenfalls vorgesehen, hier aber nicht dargestellt.) Der Beinablagebereich 27 kann mehrteilig ausgebildet sein. Der Beinablagebereich 27 ist im Bezug zum restlichen Liegenbereich 4 schwenkbar ausgebildet. Der Beinablagebereich 27 weist insbesondere keine Segmente 6 auf. Mittels des schwenkbaren Beinablagebereiches 27 ist es möglich, die Beine des Patienten 3 während der Therapie anzuwinkeln und so entsprechend das Becken zu neigen, um die Krümmung der Wirbelsäule 30 entsprechend zu verändern.

Vorzugsweise sind die Segmente 6 einzeln in ihrer Höhe zueinander verstellbar.

Die einzelnen Segmente 6 sind vorzugsweise zumindest teilweise stufenlos höhenverstellbar. Die einzelnen Segmente 6 sind vorzugsweise zumindest teilweise stufenlos höhenverstellbar. Hierdurch ist die Wirbelsäule 30 in eine geeignete individuelle Behandlungsposition bringbar und damit der Angriffswinkel der Zugkräfte genauer einstellbar. Dies hat den Vorteil, dass die Segmente 6 - zusätzlich zu dem Hub der Stützelemente - an die Form der Wirbelsäule 30 anpassbar sind. Es ist denkbar, dass jedes Segment 6 durch ein separates Hubglied (nicht dargestellt) verstellbar ist. Dies ermöglicht eine besonders genaue Höhenverstellung aller Segmente 6. Es ist jedoch auch denkbar, dass nur einige Segmente 6 höhenverstellbar sind, wodurch weniger Hubglieder eingesetzt werden müssen, was Kosten spart.

Dadurch, dass die einzelnen Segmente 6, insbesondere stufenlos höhenverstellbar sind, können die der Trennstelle 22 benachbarten Segmente 6 im Wesentlichen in einer Höhe angeordnet werden, insbesondere im 90°-Winkel zur Wirbelkörperober- bzw. -unterkante angeordnet werden. Diese der Trennstelle 22 benachbarten Segmente 6 definieren den Therapiebereich. Dadurch, dass die der Trennstelle 22 zugeordneten Segmente 6 auf eine Höhe gebracht werden, erfolgt die Einleitung der Zugkraft immer im Wesentlichen im 90°-Winkel zur Wirbelkörperober- bzw. -unterkante. Hierdurch ist es möglich, eine bestimmte Bandscheibe oder ein bestimmtes Wirbelgelenkpaar mit zwei gleich großen, wiederholgenauen und einstellbaren Kräfte unter einem optimalen und wiederholgenau einstellbaren Kraftwinkel in entgegengesetzter Richtung zu entlasten. Durch die aktive Höhenverstellbarkeit der Segmente 6 und/oder der Stützelemente 7 kann die Wirbelsäule derart gebogen und/oder gestreckt werden, so dass die Nervenwurzelaustrittsöffnungen besonders gut erweitert bzw. geweitet werden können. Dies kann zu einer Schmerzlinderung führen. Ferner können die kleinen Wirbelkörpergelenke entsprechend gedehnt werden, was ebenfalls zu einer Schmerzlinderung beitragen kann. Durch die aktive Höhenverstellbarkeit der Segmente 6 und/oder der Stützelemente 7 kann ferner eine Mobilisation der Wirbelsäule 30 erzielt werden.

In der in Fig. 1 bis 12 dargestellten Ausgestaltung sind die Segmente 6 dazu an einer Profilschiene 32 abgestützt, wobei die Profilschiene 32 ein Höhenprofil abbildet. Die Segmente 6 sind auf der höhenprofilierten Profilschiene 32 abgestützt, wobei insbesondere durch Verschieben der Profilschiene 32 unterschiedliche Stellungen der Segmente 6 einstellbar sind. Je nach dem wie die Profilschiene 32 nun in Längsrichtung verfahren wird, werden unterschiedliche Wölbungen und Krümmungen der Hälften 8, 9 eingestellt. Beispielsweise ist in Fig. 2 dargestellt, wie die rechte Reihe 9 entsprechend durch Verfahren der Profilschiene 32 verfahren werden kann. Hierdurch ist es möglich, unterschiedliche Wölbungen einzustellen, die jeweils zur Dekompression der Lendenwirbelsäule, in der Brustwirbelsäule oder der Halswirbelsäule unterschiedlich gewählt werden können. Es ist denkbar, unterschiedliche Profilschienen 32 zu verwenden, die jeweils ein entsprechend geeignetes unterschiedliches Höhenprofil abbilden.

In der in Fig. 13 bis 20 dargestellten Ausgestaltung der Extensionsliegenvorrichtung 2 ist eine weitere Möglichkeit der zumindest teilweisen Höhenverstellung der Segmente 6 dargestellt. Hierbei weist der Liegenbereich 4 einen schwenkbaren Kopfbereich 26 mit mehreren der Segmente 6 auf. Diese dem Kopfbereich 26 zugeordneten Segmente 6 können insbesondere schmaler als die restlichen an einem entsprechenden Grundgestell (nicht näher dargestellt) angeordneten Segmente 6 sein.

In der in Fig. 22 bis 25 dargestellten Ausgestaltung der Extensionsliegenvorrichtung 36 sind mehrere Profilschienen 43, 44 und 45 vorgesehen. Die Profilschienen 43, 44 und 45 geben die Höhenpositionen der Segmente 6 vor, da die Segmente 6 auf den Profilschienen 43, 44, 45 funktional wirksam abgestützt sind. Die Profilschiene 43 ist ortsfest, d.h. Unbeweglich angeordnet und bildet eine neutrale Grundstellung der Wirbelsäule nach (vgl. Fig. 23). Die Profilschiene 43 weist dazu konvexe Wölbungen entsprechend der Lordose der Lendenwirbelsäule und der Halswirbelsäule (nicht näher bezeichnet) auf.

Diese Grundstellung der Segmente 6 ist nun mittels der beiden weiteren Profilschienen 44, 45 veränderbar, um beispielsweise den Liegenbereich 4 an die Größe des Patienten 3 anzupassen und/oder um die Therapieposition des Patienten 3 zu verändern. Die beiden Profilschienen 44, 45 sind schwenkbar und axial verschieblich. Die beiden Profilschienen 44, 45 weisen eine konvexe Krümmung auf. Durch Schwenken und/oder Verschieben der Profilschienen 44, 45 ist der Liegenbereich 4 vorzugsweise vollständig, insbesondere im Bereich der Halswirbelsäule bzw. im Bereich der Hüfte und der Lendenwirbelsäule anhebbar.

Die Profilschiene 44, 45 weisen seitliche Nuten 46 auf, wobei die Schlitten 37, 38 mit Rollen 47 in die Nuten 46 eingreifen (vgl. Fig. 25). Die Segmente 6 sind mit nicht näher bezeichneten Bereichen auf der Oberseite der Profilschienen 44, 45 abgestützt. Es sind wie in Fig. 25 gut ersichtlich jeweils paarweise Profilschienen 44, 45 seitlich des U-förmigen Schlittens 37, 38 vorgesehen, so dass die Segmente 6 jeweils beidseitig des Greifbereiches 40 abgestützt sind. Der Schlitten 37, 38 weist unterhalb der paarweise angeordneten Rollen 47 weitere Rollen 48 auf. Die Rollen 48 setzen auf eine horizontal angeordnete Schiene 50 auf, wenn die Profilschienen 44, 45 nach unten geschwenkt sind. In dieser Stellung setzen die Segmente 6 dann auf der Profilschiene 43 (Vgl. Fig. 23) auf.

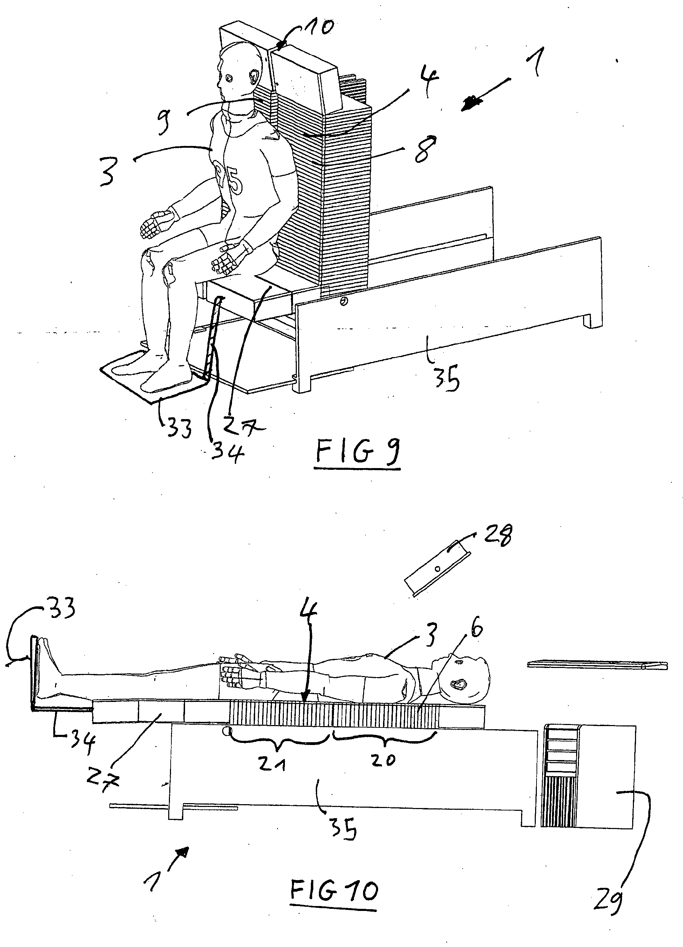

In besonders bevorzugter Ausgestaltung ist der gesamte Liegenbereich 4 zudem schwenkbar an einem Gestell 35 angeordnet, so dass der Patient 3 entweder sitzend (vgl. Fig. 9 bis 12) oder stehend (vgl. Fig. 13, 14) in die liegende Position gekippt werden kann. Dies hat den Vorteil, dass der Patient 3 nicht auf den Liegenbereich 4 gehoben werden muss, sondern dass er automatisch geschwenkt wird. Der Schwenkmechanismus weist insbesondere einen Motor zur Ausführung der Schwenkbewegung auf. Es ist für den Patienten 3 angenehmer, sich entsprechend hinzustellen oder auf den Beinablagebereich 27 zu setzen und danach in den Liegenbereich 4 zu schwenken, als dass der Patient 3 eigenhändig auf den Liegenbereich 4 mit Rückenschmerzen klettern muss. Es ist eine Fußplatte 33 vorgesehen (vgl. Fig. 9, 10, 12a, 12b), auf der der Patient 3 seine Füße abstellen kann. Die Fußplatte 33 ist über eine vorzugsweise längenverstellbare Schiene 34 mit dem Beinablagebereich 27 verbunden. Dadurch dass der Patient 3 seine Füße auf der Fußplatte 33 abstellen kann, ist die Belastung der Wirbelsäule beim Schwenken vermindert. Auch ist es für den Patienten 3 angenehmer nach der Therapie in eine sitzende oder stehende Stellung geschwenkt zu werden als sich selbstständig aus der liegenden Position zu erheben.

Ferner ist es denkbar, zusätzliche Mittel - ein Wärmemittel, ein Gleichstrommittel und/oder ein Massagemittel, insbesondere ein Vibrationsmittel - einzusetzen, die die oberflächliche oder tiefere Muskulatur des Patienten 3 entspannt. In bevorzugter Ausgestaltung wird die Wärme und/oder eine Massage über eine Vibration oder dergleichen über die Stützelemente 7 eingebracht. Die Stützelemente 7 können leitend ausgebildet sein. Hierdurch kann eine Gleichstrombehandlung erfolgen.

Die eingeleiteten Zugkräfte und die entsprechend auftretenden Gegenzugkräfte können nun zeitlich konstant, linear ansteigend, in Intervallen stufenweise ansteigend und/oder parabelförmig ansteigend appliziert werden. Nach einer ansteigenden Phase werden die Zugkräfte in einer abfallenden Phase wieder reduziert. Die Reduzierung kann linear abfallend, stufenweise abfallend, hyperbelförmig abfallend oder parabelförmig abfallend erfolgen. In bevorzugter Ausgestaltung werden die Zugkräfte und oder die Gegenzugkräfte dem Patienten 3 während der Behandlung auf dem Monitor 28 dargestellt.

Vorzugsweise sind Sensoren (nicht dargestellt) vorgesehen. Die Sensoren können in den Liegenbereich 4 integriert sein, bspw. können als Sensoren Oberflächenelektroden eingesetzt werden. Vorzugsweise sind mindestens zwei Oberflächenelektroden, vorzugsweise vier Oberflächenelektroden, zur Messung der Muskelspannung vorgesehen. Diese Oberflächenelektroden messen die Muskelspannung des Patienten 3, insbesondere die Muskelspannung in der Rückenmuskulatur. Durch ein Biofeedback-Verfahren kann der Patient 3 während der Behandlung lernen, dass die gemessene muskuläre Gegenspannung zum Therapieende hin abnimmt. Die eingeleiteten, möglicherweise unterschiedlichen Zugkräfte und/oder die Sensormesswerte werden dem Patienten 3 auf einem Monitor 28 visuell dargestellt. Der Monitor 28 ist an einen Computer 29 angeschlossen. Durch die Sensoren und die Darstellung in der entsprechenden gemessenen Muskelspannung wird dem Patienten 3 eine Möglichkeit gegeben, gezielt zu lernen, die Muskulatur zu entspannen und hiermit die durch die Muskulatur aufgebrachte Gegenspannung zu minimieren.

Insgesamt ist mit den Extensionsliegenvorrichtungen 1, 2, 36 eine präzisere, effektivere und individuellere Therapie ermöglicht.

Die eingangs genannten Nachteile sind daher vermieden und entsprechende Vorteile sind erzielt.

### Bezugszeichenliste:

- 1: Extensionsliegenvorrichtung
- 2: Extensionsliegenvorrichtung
- 3: Patient
- 4: Liegenbereich
- 5: Antriebssystem
- 6: Segmente
- 7: Stützelement
- 8: Hälfte
- 9: Hälfte
- 10: Spalt
- 11: Rahmen
- 12: Druckluftanschluss
- 13: Zylinder
- 14: Matrix
- 15: Auflage
- 16: Antriebsmotor
- 17: Antriebsmotor
- 18: Antriebsmotor
- 19: Antriebsmotor
- 20: erste Segmentgruppe
- 21: zweite Segmentgruppe
- 22: Trennstelle
- 23: Koppelmittel
- 24: Koppelmittel
- 25a: Bolzenaufnahme
- 25b: Bolzen
- 26: Kopfbereich
- 27: Beinablagebereich
- 28: Monitor
- 29: Computer
- 30: Wirbelsäule
- 31: Wirbelkörper
- 32: Profilschiene
- 33: Fußplatte
- 34: Schiene
- 35: Gestell
- 36: Extensionsliegenvorrichtung
- 37: Schlitten
- 38: Schlitten
- 40: Greifbereich
- 41: Gegenhaltplatte
- 42: Gegenhaltplatte
- 43: Profilschiene
- 44: Profilschiene
- 45: Profilschiene

## Patentansprüche

1. Extensionsliegenvorrichtung (1, 2, 36) zur Dekompression der Wirbelsäule (30) und/oder zur Dekompression von Gelenken eines Patienten (3), mit einem Liegenbereich (4) und mit einem Antriebssystem (5), wobei der Liegenbereich (4) mehrere Segmente (6) aufweist, wobei der Patient (3) auf dem Liegenbereich (4) anordbar ist, wobei zumindest eines der Segmente (6) mit dem Antriebssystem (5) derart bewegbar ist, so dass zur Dekompression eine Zugkraft und eine Gegenzugkraft auf den Patienten (3) ausübbar ist, **dadurch gekennzeichnet, dass** die Segmente (6) mehrere aufragende Stützelemente (7) aufweisen, wobei der Patient (3) zumindest teilweise von den Stützelementen (7) abstützbar ist, wobei die Stützelemente (7) in unterschiedlicher Höhe derart anordbar sind, so dass die Anordnung der Stützelemente (7) der Kontur des Patienten (3) in Längsrichtung und Querrichtung anpassbar ist, wodurch der Patient (3) in einem mittels der Stützelemente (7) gebildeten Abdruck gehalten ist, wobei der Liegenbereich (20) wahlweise an unterschiedlichen Trennstellen (22) zwischen benachbarten Segmenten (6) in eine erste Segmentgruppe (20) und in eine zweite Segmentgruppe (21) teilbar ist, wobei die erste Segmentgruppe (20) und die zweite Segmentgruppe (21) jeweils mindestens ein Segment (6) aufweisen, wobei ein der Trennstelle (22) benachbartes Segment (6) der ersten Segmentgruppe (20) und ein der Trennstelle (22) benachbartes Segment (6) der zweiten Segmentgruppe (21) mittels des Antriebssystems (5) zur Dekompression relativ zueinander verfahrbar sind.

2. Extensionsliegenvorrichtung nach Anspruch 1, **dadurch gekennzeichnet, dass** die Stützelemente (7) im wesentlichen stiftförmig ausgebildet sind, wobei die Stützelemente in einem aufragenden Zylinder (13) verschieblich angeordnet ist, wobei die Stützelement (7) mittels mindestens eines Aktors, insbesondere pneumatisch verfahrbar sind oder mittels mehrerer Federmittel abgestützt sind.

3. Extensionsliegenvorrichtung nach einem der vorstehenden Ansprüche, **dadurch gekennzeichnet, dass** das Antriebssystem (5) verfahrbare Koppelmittel (23, 24) aufweist, wobei die Koppelmittel (23, 24) wahlweise mit jeweils der Trennstelle (22) benachbarten Segmenten (6) koppelbar sind.

4. Extensionsliegenvorrichtung nach einem der vorstehenden Ansprüche, **dadurch gekennzeichnet, dass** die Segmente (6) zumindest teilsweise vorzugsweise stufenlos höhenverstellbar sind.

5. Extensionsliegenvorrichtung nach einem der vorstehenden Ansprüche, **dadurch gekennzeichnet, dass** die Segmente (6) auf mindestens einer höhenprofilierten Profilschiene (32, 43, 44, 45) abgestützt sind, wobei insbesondere durch Verschieben und/oder Verschwenken der Profilschiene (32, 44, 45) unterschiedliche Stellungen der Segmente (6) einstellbar sind.

6. Extensionsliegenvorrichtung nach einem der vorstehenden Ansprüche, **dadurch gekennzeichnet, dass** mehr als 10 Stützelemente (6), insbesondere mehr als 100 Stützelemente (6) vorgesehen sind.

7. Extensionsliegenvorrichtung nach einem der vorstehenden Ansprüche, **dadurch gekennzeichnet, dass** unterschiedliche Segmente (6) eine unterschiedlichen Längsausdehnung - in Längsrichtung des Liegenbereiches (4) gemessen - aufweisen, wobei insbesondere die zur Ablage der Halswirbelsäule dienenden Segmente (6) eine kleinere Ausdehnung in Längsrichtung aufweisen als die zur Abstützung des Lendenwirbelbereiches und/oder des Brustwirbelbereiches dienenden Segmente (6).

8. Extensionsliegenvorrichtung nach einem der vorstehenden Ansprüche, **dadurch gekennzeichnet, dass** der Liegenbereich (4) zwei Hälften (8, 9) aufweist, wobei jede der Hälften (8, 9) mehrere Segmente (6) aufweist, die jeweils in eine entsprechende obere Segmentgruppe (20) und eine untere Segmentgruppe (21) aufteilbar sind, wobei jeder Reihe (8, 9) mindestens ein Antriebsmotor (16, 17; 18, 19) zugeordnet ist.

9. Extensionsliegenvorrichtung nach einem der vorstehenden Ansprüche, **dadurch gekennzeichnet, dass** mindestens ein Sensor, insbesondere mindestens zwei Oberflächenelektroden zur Messung einer Muskelspannung vorgesehen ist, wobei vorzugsweise die gemessene Muskelspannung den Patienten über einen Monitor (28) darstellbar ist.

10. Extensionsliegenvorrichtung nach einem der vorstehenden Ansprüche, **dadurch gekennzeichnet, dass** der Liegenbereich (4) ein Wärmemittel, ein Gleichstrommittel und/oder ein Massagemittel, insbesondere ein Vibrationsmittel zur Muskelentspannung des Patienten (3) aufweist.

11. Extensionsliegenvorrichtung nach einem der vorstehenden Ansprüche, **dadurch gekennzeichnet, dass** mindestens zwei aufblasbare Kammern seitlich des Patienten (3) vorgesehen sind, so dass der Patient (3) durch Aufblasen der Kammern in dem entstehenden Abdruck haltbar ist.

12. Extensionsliegenvorrichtung nach einem der vorstehenden Ansprüche, **dadurch gekennzeichnet, dass** der Liegenbereich (4) schwenkbar an einem Gestell (35) angeordnet, so dass der Patient (3) sitzend oder stehend in die liegende Position schwenkbar ist.

## Claims

1. Extension bed device (1, 2, 36) for decompressing the spine (30) and/or for decompressing joints of a patient (3), having a bed area (4) and having a drive system (5), wherein the bed area (4) has several segments (6), wherein the patient (3) is arrangeable on the bed area (4), wherein at least one of the segments (6) is movable by the drive system (5) such that, for decompression, traction and counter-traction are able to be exerted on the patient (3), **characterized in that** the segments (6) have several protruding support elements (7), wherein the patient (3) is able to be supported at least partially by the support elements (7), wherein the support elements (7) are arrangeable at different heights such that the arrangement of the support elements (7) is adaptable to the contour of the patient (3) in the longitudinal direction and transverse direction, with the result that the patient (3) is held in an impression formed by means of the support elements (7), wherein the bed area (20) is divisible selectively at different separating points (22) between adjacent segments (6) into a first segment group (20) and into a second segment group (21), wherein the first segment group (20) and the second segment group (21) each have at least one segment (6), wherein a segment (6), next to the separating point (22), of the first segment group (20) and a segment (6), next to the separating point (22), of the second segment group (21) are movable relative to one another by means of the drive system (5) for decompression.

2. Extension bed device according to Claim 1, **characterized in that** the support elements (7) are formed in a substantially pin-like manner, wherein the support elements are arranged in a displaceable manner in a protruding cylinder (13), wherein the support elements (7) are movable, in particular pneumatically, by means of at least one actuator or are supported by means of several spring means.

3. Extension bed device according to either of the preceding claims, **characterized in that** the drive system (5) has movable coupling means (23, 24), wherein the coupling means (23, 24) are selectively couplable to segments (6) respectively next to the separating point (22).

4. Extension bed device according to one of the preceding claims, **characterized in that** the segments (6) are at least partially height-adjustable preferably in an infinitely variable manner.

5. Extension bed device according to one of the preceding claims, **characterized in that** the segments (6) are supported on at least one height-profiled profile rail (32, 43, 44, 45), wherein different positions of the segments (6) are settable in particular by displacing and/or pivoting the profile rail (32, 44, 45).

6. Extension bed device according to one of the preceding claims, **characterized in that** more than 10 support elements (6), in particular more than 100 support elements (6) are provided.

7. Extension bed device according to one of the preceding claims, **characterized in that** different segments (6) have different longitudinal extensions - as measured in the longitudinal direction of the bed area (4) - wherein in particular the segments (6) that serve for receiving the cervical spine have a smaller extension in the longitudinal direction than the segments (6) that serve for supporting the lumbar spine and/or the thoracic spine.

8. Extension bed device according to one of the preceding claims, **characterized in that** the bed area (4) has two halves (8, 9), wherein each of the halves (8, 9) has several segments (6), which are each subdivisible into a corresponding upper segment group (20) and a lower segment group (21), wherein each row (8, 9) is assigned at least one drive motor (16, 17; 18, 19).

9. Extension bed device according to one of the preceding claims, **characterized in that** at least one sensor, in particular at least two surface electrodes for measuring muscle tension are provided, wherein preferably the measured muscle tension is able to be displayed to the patient via a monitor (28).

10. Extension bed device according to one of the preceding claims, **characterized in that** the bed area (4) has a heating means, a DC means and/or a massaging means, in particular a vibratory means for relaxing the muscles of the patient (3).

11. Extension bed device according to one of the preceding claims, **characterized in that** at least two inflatable chambers are provided to the sides of the patient (3), such that the patient (3) is able to be held in the produced impression by inflation of the chambers.

12. Extension bed device according to one of the preceding claims, **characterized in that** the bed area (4) is arranged in a pivotable manner on a frame (35), such that the sitting or standing patient (3) is pivotable into the lying position.

## Revendications

1. Dispositif d'extension en décubitus (1, 2, 36) pour la décompression de la colonne vertébrale (30) et/ou pour la décompression d'articulations d'un patient (3), comprenant une zone de couchage (4) et un système d'entraînement (5), la zone de couchage (4) comprenant plusieurs segments (6), le patient (3) pouvant être disposé sur la zone de couchage (4), au moins l'un des segments (6) pouvant être déplacé à l'aide du système d'entraînement (5) de telle sorte que, pour la décompression, une force de traction et une force antagoniste à la traction puissent être exercées sur le patient (3), **caractérisé en ce que** les segments (6) comprennent plusieurs éléments de support (7) saillants, le patient (3) pouvant être supporté au moins partiellement par les éléments de support (7), les éléments de support (7) pouvant être disposés à des hauteurs différentes de telle sorte que l'agencement des éléments de support (7) puisse être adapté aux contours du patient (3) dans la direction longitudinale et dans la direction transversale, de sorte que le patient (3) soit retenu dans une empreinte formée au moyen des éléments de support (7), la zone de couchage (20) pouvant être divisée sélectivement, en différents points de séparation (22) entre des segments adjacents (6), en un premier groupe de segments (20) et en un deuxième groupe de segments (21), le premier groupe de segments (20) et le deuxième groupe de segments (21) comprenant respectivement au moins un segment (6), un segment (6), adjacent au point de séparation (22), du premier groupe de segments (20) et un segment (6), adjacent au point de séparation (22), du deuxième groupe de segments (21) pouvant être déplacés l'un par rapport à l'autre au moyen du système d'entraînement (5) en vue de la décompression.

2. Dispositif d'extension en décubitus selon la revendication 1, **caractérisé en ce que** les éléments de support (7) sont réalisés sensiblement en forme de tiges, les éléments de support étant disposés de manière mobile dans un cylindre (13) saillant, les éléments de support (7) étant déplaçables en particulier de manière pneumatique au moyen d'au moins un actionneur, ou étant supportés grâce à plusieurs moyens ressorts.

3. Dispositif d'extension en décubitus selon l'une quelconque des revendications précédentes, **caractérisé en ce que** le système d'entraînement (5) comprend des moyens d'accouplement déplaçables (23, 24), les moyens d'accouplement (23, 24) pouvant être accouplés sélectivement respectivement au point de séparation (22) de segments adjacents (6).

4. Dispositif d'extension en décubitus selon l'une quelconque des revendications précédentes, **caractérisé en ce que** les segments (6) sont réglables en hauteur au moins partiellement, de préférence de manière continue.

5. Dispositif d'extension en décubitus selon l'une quelconque des revendications précédentes, **caractérisé en ce que** les segments (6) sont supportés sur au moins un rail profilé (32, 43, 44, 45) profilé en hauteur, différentes positions des segments (6) pouvant être réglées en particulier par coulissement et/ou pivotement du rail profilé (32, 44, 45).

6. Dispositif d'extension en décubitus selon l'une quelconque des revendications précédentes, **caractérisé en ce que** plus de 10 éléments de support (6), en particulier plus de 100 éléments de support (6) sont prévus.

7. Dispositif d'extension en décubitus selon l'une quelconque des revendications précédentes, **caractérisé en ce que** différents segments (6) présentent une étendue longitudinale différente, mesurée dans la direction longitudinale de la zone de couchage (4), les segments (6) servant à la réception de la colonne cervicale présentant en particulier une étendue plus petite dans la direction longitudinale que les segments (6) servant au support de la zone des vertèbres lombaires et/ou de la zone des vertèbres dorsales.

8. Dispositif d'extension en décubitus selon l'une quelconque des revendications précédentes, **caractérisé en ce que** la zone de couchage (4) comprend deux moitiés (8, 9), chacune des moitiés (8, 9) comprenant plusieurs segments (6) qui peuvent être répartis respectivement en un groupe de segments supérieur correspondant (20) et un groupe de segments inférieur (21), au moins un moteur d'entraînement (16, 17 ; 18, 19) étant associé à chaque rangée (8, 9).

9. Dispositif d'extension en décubitus selon l'une quelconque des revendications précédentes, **caractérisé en ce qu'**au moins un capteur, en particulier au moins deux électrodes de surface, sont prévus pour mesurer une tension musculaire, la tension musculaire mesurée étant de préférence présentée au patient par le biais d'un écran (28).

10. Dispositif d'extension en décubitus selon l'une quelconque des revendications précédentes, **caractérisé en ce que** la zone de couchage (4) comprend un moyen de chauffage, un moyen à tension continue et/ou un moyen de massage, en particulier un moyen de génération de vibrations servant à la détente musculaire du patient (3).

11. Dispositif d'extension en décubitus selon l'une quelconque des revendications précédentes, **caractérisé en ce qu'**au moins deux chambres gonflable sont prévues sur les côtés du patient (3), de telle sorte que le patient (3) puisse être retenu, par gonflage des chambres, dans l'empreinte se formant.

12. Dispositif d'extension en décubitus selon l'une quelconque des revendications précédentes, **caractérisé en ce que** la zone de couchage (4) est disposée de manière pivotante sur un cadre (35), de telle sorte que le patient (3) puisse être pivoté, assis ou debout, dans la position couchée.
